# EUROPEAN PATENT SPECIFICATION

(11) **EP 3 490 999 B1**
(45) Date of publication and mention of the grant of the patent: **14.04.2021**
(21) Application number: 17754872.4
(22) Date of filing: 27.07.2017
(51) Int. Cl.: C07F 9/50, C07C 43/13, C07D 403/10, C07C 215/52, C07C 323/13, C07D 209/86, C07F 7/00, C08F 4/64, C07C 43/23, C07C 215/74, C07C 217/18, C07C 323/12, C07C 323/18

(54) **SYNTHESIS OF M-TERPHENYL PENDANT BIS-ETHER LIGANDS AND METAL COMPLEX AND THEIR USE IN OLEFIN POLYMERIZATION**
SYNTHESE VON AN M-TERPHENYL HÄNGENDEN BIS-ETHERLIGANDEN UND METALLKOMPLEX UND DEREN VERWENDUNG BEI DER OLEFINPOLYMERISIERUNG
SYNTHÈSE DE LIGANDS BIS-ÉTHER PENDANTS M-TERPHÉNYLE, COMPLEXES MÉTALLIQUES ET LEUR UTILISATION DANS LA POLYMÉRISATION D'OLÉFINES

(30) Priority: 29.07.2016 US 201662368699 P
(43) Date of publication of application: 05.06.2019
(73) Proprietor: Dow Global Technologies LLC, Midland, MI 48674 (US)
(72) Inventor: OBER, Matthias, Stefan, Midland MI 48667 (US); ROMER, Duane R., Auburn MI 48611 (US); IVERSON, Carl Nicholas, Los Alamos NM 67544 (US); MARGL, Peter Michael, Midland MI 48667 (US)
(74) Representative: Boult Wade Tennant LLP
(86) International application number: PCT/US2017/044156
(87) International publication number: WO 2018/022871

(56) References cited:
- WO-A1-2008/036882
- STEPHEN I. KLINK ET AL: "Near-Infrared and Visible Luminescence from Terphenyl-Based Lanthanide(III) Complexes Bearing Amido and Sulfonamido Pendant Arms", EUROPEAN JOURNAL OF ORGANIC CHEMISTRY, vol. 2000, no. 10, 1 May 2000 (2000-05-01) , pages 1923-1931, XP055406484, DE ISSN: 1434-193X, DOI: 10.1002/(SICI)1099-0690(200005)2000:10<192 3::AID-EJOC1923>3.0.CO;2-W
- CATHLEEN M. UNRAU ET AL: "Directed ortho metalation - suzuki cross coupling connections. Convenient regiospecific routes to functionalized m- and p-teraryls and m-quinquearyls", TETRAHEDRON LETTERS, vol. 33, no. 20, 1 May 1992 (1992-05-01), pages 2773-2776, XP055406483, AMSTERDAM, NL ISSN: 0040-4039, DOI: 10.1016/S0040-4039(00)78854-X
- GUY A. EDOUARD ET AL: "Aryl Ether Cleavage by Group 9 and 10 Transition Metals: Stoichiometric Studies of Selectivity and Mechanism", ORGANOMETALLICS, vol. 34, no. 21, 9 November 2015 (2015-11-09), pages 5254-5277, XP055406488, US ISSN: 0276-7333, DOI: 10.1021/acs.organomet.5b00710
- DATABASE WPI Week 201581 2015 Thomson Scientific, London, GB; AN 2015-680531 XP002773987, & JP 2015 193612 A (MITSUI CHEM INC) 5 November 2015 (2015-11-05) -& JP 2015 193612 A (MITSUI CHEMICALS INC) 5 November 2015 (2015-11-05)

## Description

### Technical Background

Embodiments of the present disclosure generally relate to the synthesis of *m-*terphenyl pendant bis-ether ligands and transition metal complexes used in olefin polymerization.

### Background

Olefin based polymers are utilized in the manufacture of a variety of articles and products, thus there is a high industrial demand for such polymers. Olefin based polymers, such as polyethylene and/or polypropylene, are produced via various catalyst systems. Selection of such catalyst systems used in the polymerization process is an important factor contributing to the characteristics and properties of such olefin based polymers.

Some olefin polymerization catalysts based on group IV metals are known, but the catalyst systems for producing polyethylene typically comprise a chromium based catalyst system, a Ziegler Natta catalyst system, and a molecular (either metallocene or non-metallocene) catalyst system. Most of these catalysts are expensive, not easily adaptable, or ineffective on an industrial scale.

STEPHEN I. KLINK ET AL, "Near-Infrared and Visible Luminescence from Terphenyl-Based Lanthanide(III) Complexes Bearing Amido and Sulfonamido Pendant Arms", EUROPEAN JOURNAL OF ORGANIC CHEMISTRY, DE, (20000501), vol. 2000, no. 10 describes a series of *m*-terphenyl-based ligands bearing three coordinating oxyacetate and two amido or two sulfonamido groups, (**1a-b**)H₃ and (**2**)H₃, respectively, that have been synthesized and characterized.

CATHLEEN M. UNRAU ET AL, "Directed ortho metalation - suzuki cross coupling connections. Convenient regiospecific routes to functionalized m- and p-teraryls and m-quinquearyls", TETRAHEDRON LETTERS, AMSTERDAM, NL, (19920501), vol. 33, no. 20 describes rapid access to a variety of substituted m- and p- teraryls and m-quinquearyls via Pd-catalyzed, one-pot, sequential and 1:2 cross coupling of aryl halides with aryl boronic acids, both available by metal-halogen exchange or directed ortho metalation tactics.

GUY A. EDOUARD ET AL, "Aryl Ether Cleavage by Group 9 and 10 Transition Metals: Stoichiometric Studies of Selectivity and Mechanism", ORGANOMETALLICS, US, (20151109), vol. 34, no. 21, doi:10.1021/acs.organomet.5b00710, ISSN 0276-7333, pages 5254 - 5277 describes investigation of the reactivity of terphenyl diphosphines bearing arylmethyl ether or aryl-aryl ether moieties with M⁰ (M = Ni, Pd, Pt), M'^{I} (M' = Co, Rh, Ir), or M^{II} centers to gain mechanistic insight into intramolecular aryl-ether bond cleavage in structurally related metal complexes.

DATABASE WPI, 0, Derwent World Patents Index, (2015), vol. 2015, no. 81, Database accession no. 2015-680531, XP002773987 & JP2015193612 A 20151105 (MITSUI CHEM INC) [A] 1-16 describes a new transition metal compound used for olefin polylmerization catalyst in manufacture of olefin polymer .

WO2008036882 describes a non-metallocene organometallic complex comprising a tridentate ligand and a metal bonded to a tridentate ligand.

Despite the research efforts in developing catalyst systems suitable for polyolefin polymerization, such as polyethylene, there is still a need for improved polymerization catalysts to meet industrial demand for olefin based polymers.

Accordingly, the present embodiments are directed to catalyst systems, which provide alternative synthetic schemes for meeting industrial demand of olefin based polymers. Embodiments of the present disclosure are directed to new procatalysts and the corresponding ligand frameworks that incorporate a tetradentate ligand, having dianionic donor properties, with a triaryle backbone with a bridging component. The procatalyst and the ligand framework have a high capacity for functional group variation, and an ease of synthesis to facilitate scaling up to commercial scale production. In addition, the structure of these molecules offers the ability to synthesize a structural motif similar to those found in the highly active bisphenylphenol systems. One advantage of this synthetic scheme is that by altering the starting materials the substituent groups on the aryl backbone, the coordinating atoms in the ligand, and the bridge can be easily and systematically changed. This synthetic scheme offers a wide array of variation within this system, and the ability to optimize the catalytic properties. By utilizing simple starting materials and reagents already known, a previously unknown procatalyst and ligand framework was surprisingly synthesized.

One embodiment of this disclosure is a metal complex comprising the following structure: 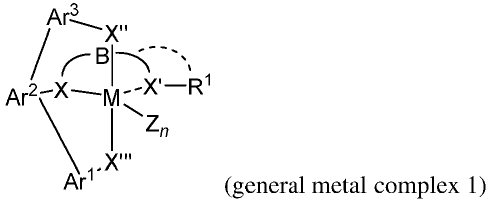 wherein at least two of the following bonds X-M, X' -M, X" -M, and X'" -M are covalent and at least one is dative. A covalent or dative bond between M and X' is optional. The Ar¹, Ar², and Ar³ are independently an aryl moiety or heteroaryl moiety. B is an aliphatic moiety or heteroaliphatic moiety linking X and X', and has from one to 50 atoms, not counting hydrogen atoms. X is oxygen, sulfur, substituted nitrogen, substituted phosphorus, or substituted carbon; X' is oxygen, sulfur, nitrogen, substituted nitrogen, substituted phosphorus, or a substituted carbon group. X" and X'" selected from oxygen, sulfur, substituted nitrogen, or substituted phosphorus. R¹ is either: 1) an aliphatic moiety, a heteroaliphatic moiety, an aromatic moiety, or a heteroaromatic moiety; or 2) part of an aliphatic carbocycle, an aromatic carbocycle, a heterocyclic moiety, or a heteroaromatic moiety. M is one or more metals selected from a Group 4 element;
wherein the terms "substituted nitrogen" is a tertiary, secondary or primary nitrogen moiety and "substituted phosphorus" is a tertiary, secondary or primary phosphorous moiety; and
substituted carbon is at least one hydrogen atom bound to a carbon atom is replaced with one or more substituents that are functional groups selected from hydroxyl, alkoxyl, alkylthio, amino, halo and silyl.

Another embodiment of the disclosure is directed to a terphenyl ligand comprising a terphenyl ligand according to the following structure 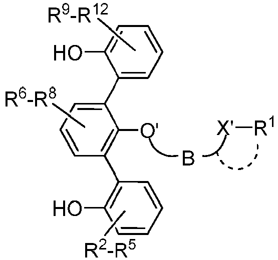 wherein
B is an aliphatic moiety or heteroaliphatic moiety linking O' and X', wherein B has from one to 50 atoms, not counting H; and
B has one to 10 atoms on shortest linkage path between O' and X', not counting H or side groups;
X' is oxygen, sulfur, substituted nitrogen, substituted phosphorus, or a substituted carbon;
R¹ is either: 1) an aliphatic moiety, a heteroaliphatic moiety, an aromatic moiety, or a heteroaromatic moiety; or 2) part of an aliphatic carbocycle, an aromatic carbocycle, a heterocyclic moiety, or a heteroaromatic moiety; and
R²-R¹² include the same or different moieties selected from aromatic moieties, aliphatic moieties, heteroaromatic moieties, heteroaliphatic moieties, heteroatoms, substituted heteroatoms, or halide moieties;
wherein the terms "substituted nitrogen" is a tertiary, secondary or primary nitrogen moiety and "substituted phosphorus" is a tertiary, secondary or primary phosphorous moiety; and
the term "substituted carbon" is wherein at least one hydrogen atom bound to said carbon atom is replaced with one or more substituents that are functional groups selected from hydroxyl, alkoxyl, alkylthio, amino, halo and silyl.

### Brief Description of the Drawings

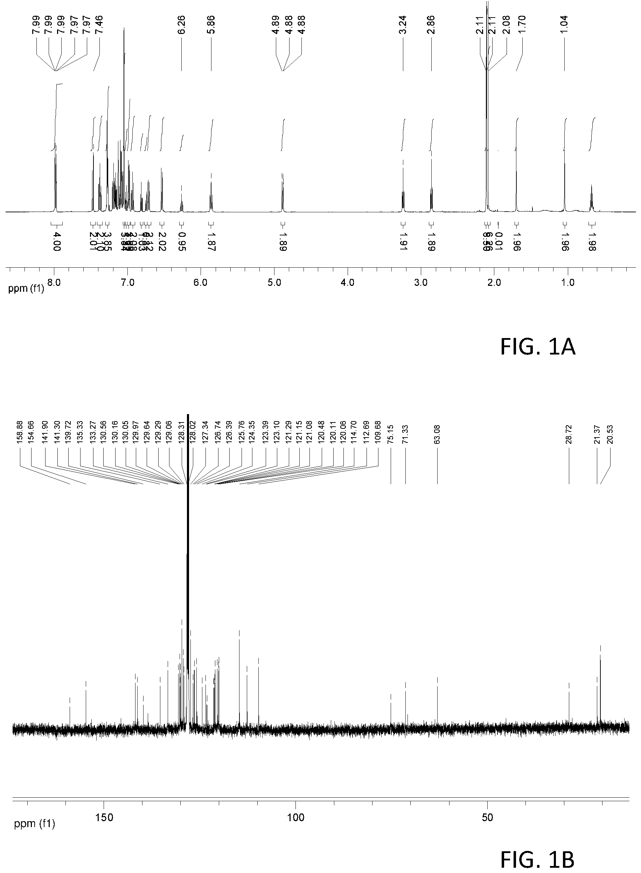

FIG. 1A is a proton NMR (nuclear magnetic resonance) spectra of Hafnium Complex 10 (L10-Hf), a metal complex embodiment discussed further below.
FIG. 1B is a carbon NMR spectra of L10-Hf.
FIG. 2 is a segment of a proton NMR spectra depicting selected signals and their corresponding protons in the Hafnium Complex 10.
FIG. 3 is a proton NMR spectra depicting a few proton NMR signals and their corresponding protons in the metal complex L10-Hf.
FIG. 4 is a graph of Gel Permeation Chromatography (GPC) results of a molecular weight distribution overlay from single polymer synthesized using 2.0 micromoles (µmol) of catalyst system L10-Hf.
FIG. 5A is an illustration of the crystal structure of the catalyst system L10-Hf, specifically showing the arrangement of the metal center in the metal complex.
FIG. 5B is an illustration of the crystal structure of the catalyst system L10-Hf, specifically showing the arrangement of the metal center in the metal complex.

### Detailed Description

Specific embodiments of the present application will now be described. The disclosure may, however, be embodied in different forms and should not be construed as limited to the embodiments set forth in this disclosure. Rather, these embodiments are provided so that this disclosure will be thorough and complete, and will fully convey the scope of the subject matter to those skilled in the art.

The term "independently selected" is used herein to indicate that the R groups, such as, R¹, R², R³, R⁴, and R⁵ can be identical or different (e.g. R¹, R², R³, R⁴, and R⁵ may all be substituted alkyls or R¹ and R² may be a substituted alkyl and R³ may be an aryl, etc.). Use of the singular includes use of the plural and vice versa (e.g., a hexane solvent, includes hexanes). A named R group will generally have the structure that is recognized in the art as corresponding to R groups having that name. These definitions are intended to supplement and illustrate, not preclude, the definitions known to those of skill in the art.

The terms "moiety," "group," and "functional group" are generally used interchangeably in this specification, but those of skill in the art may recognize certain parts of a complex or compound as being a moiety rather than a functional group and vice versa. Additionally, the term "complex" is when a metal and ligand coordinate to form a single molecular compound. For the purposes of illustration, certain representative groups are defined within this disclosure. These definitions are intended to supplement and illustrate, not preclude, the definitions known to those of skill in the art.

The term "aromatic" is used in its usual sense. The term "aryl" as used herein, and unless otherwise specified, refers to an aromatic substituent containing a single aromatic ring or multiple aromatic rings that are fused together, linked covalently, or linked to a common group such as a methylene or ethylene moiety. More specific aryl groups contain one aromatic ring or two or three fused or linked aromatic rings, e.g., phenyl, naphthyl, biphenyl, anthracenyl, phenanthrenyl, and the like. In particular embodiments, aryl substituent groups have 1 to about 200 carbon atoms, typically 1 to about 50 carbon atoms, and specifically 1 to about 20 carbon atoms. "Substituted aryl" refers to an aryl moiety substituted with one or more substituent groups, (such as tolyl, mesityl and perfluorophenyl) and the terms "heteroatom-containing aryl" and "heteroaryl" refer to aryl in which at least one carbon atom is replaced with a heteroatom (rings such as, thiophene, pyridine, isoxazole, pyrazole, pyrrole, furan or benzo-fused analogues of these rings are included in the term "heteroaryl"). In some embodiments herein, multi-ring moieties are substituent groups and in such an embodiment the multi-ring moiety can be attached at an appropriate atom. For example, "naphthyl" can be 1-naphthyl or 2-naphthyl; "anthracenyl" can be 1-anthracenyl, 2-anthracenyl or 9-anthracenyl; and "phenanthrenyl" can be 1-phenanthrenyl, 2-phenanthrenyl, 3-phenanthrenyl, 4-phenanthrenyl or 9-phenanthrenyl.

The term "aliphatic" means a non-aromatic saturated or unsaturated straight or branched hydrocarbon radical of from 1-40 carbon atoms or from 1 to 18 carbon atoms.

By "substituted" as in "substituted aliphatic moiety," "substituted aryl," "substituted alkyl," "substituted alkenyl" and the like, as alluded to in some of the aforementioned definitions, is meant that in the hydrocarbyl, hydrocarbylene, alkyl, alkenyl, aryl or other moiety, at least one hydrogen atom bound to a carbon atom is replaced with one or more substituents that are functional groups such as hydroxyl, alkoxy, alkylthio, amino, halo, silyl, and the like. When the term "substituted" appears prior to a list of possible substituted groups, it is intended that the term apply to every member of that group. That is, the phrase "substituted alkyl, alkenyl and alkynyl" is to be interpreted as "substituted alkyl, substituted alkenyl and substituted alkynyl." Similarly, "optionally substituted alkyl, alkenyl and alkynyl" is to be interpreted as "optionally substituted alkyl, optionally substituted alkenyl and optionally substituted alkynyl." However, the terms "substituted nitrogen" and "substituted phosphorous" include tertiary, secondary and primary nitrogen and phosphorous moieties.

The term "saturated" means lacking carbon-carbon double bonds, carbon-carbon triple bonds, and (in heteroatom-containing groups) carbon-nitrogen, carbon-phosphorous, and carbon-silicon double bonds. Where a saturated chemical group is substituted by one or more substituent groups, one or more double and/or triple bonds optionally may or may not be present in substituent groups. The term "unsaturated" means containing one or more carbon-carbon double bonds, carbon-carbon triple bonds, and (in heteroatom-containing groups) carbon-nitrogen, carbon-phosphorous, and carbon-silicon double bonds, not including any such double bonds that may be present in substituent groups, for example, R or X, if any, or in (hetero)aromatic rings, if any.

When a variable group, such as Ar, R, X, is defined as an element, such as oxygen or nitrogen, then that variable group is an atom that has at least as many bonds as designated by the variable and the complex. Some atoms may form more bonds than designated by the location of the variable within the complex. If this occurs, then the atom is bonded to a hydrogen atom or is substituted with an alkyl group.

The terms "halogen" or "halogen atom" mean fluorine atom (F), chlorine atom (CI), bromine atom (Br), or iodine atom (I). Each halogen atom independently is the Br, F, or Cl radical. The terms "halide" or "halo" mean fluoride (F⁻), chloride (Cl⁻), bromide (Br⁻), or iodide (I⁻) anion.

The term "heteroatom," "heteroaromatic," "heteroaryl," "heterocyclic" or "heteroaliphatic" refers to a molecule or molecular framework in which one or more carbon atoms is replaced with an atom other than carbon or hydrogen, such as nitrogen, oxygen, sulfur, phosphorus, boron or silicon. For example, a carbon in an aromatic ring is replaced by a nitrogen atom to form an aromatic ring system called pyridine, which is heteroaromatic. Other possible substitution for carbon atoms are oxygen, sulfur, phosphorus, boron or silica.

In one embodiment, a metal complex used in the catalyst systems of the present disclosure comprises the following structure: 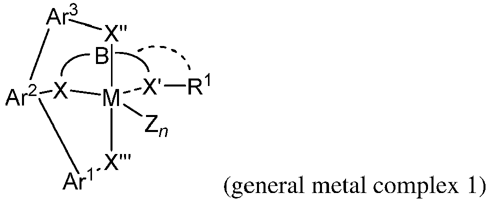 is synthesized from a terphenyl ligand comprising the following structure:

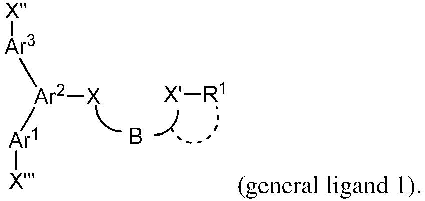

In one embodiment of the general metal complex 1, at least two of the following bonds X-M, X"-M, and X"'-M are covalent and at least one is dative. The X'-M bond is covalent, dative, or does not exist. In another embodiment of general metal complex 1 and general ligand 1, the Ar¹, Ar², and Ar³ groups are independently selected from aryl or heteroaryl moieties, in which the aryl groups or heteroaryl group may comprise two aryl or heteroaryl rings fused together. However, Ar¹ and Ar² or Ar² and Ar³ are not fused together and are connected by a single covalent bond. In one embodiment, B is an aliphatic moiety or heteroaliphatic moiety linking X and X', wherein B has from one to 50 atoms, not counting H. X is oxygen, sulfur, substituted nitrogen, substituted phosphorus, or substituted carbon. X' is oxygen, sulfur, substituted nitrogen, substituted phosphorus, or an unsaturated substituted carbon group. X" and X'" include the same or different moieties selected from oxygen, sulfur, substituted nitrogen, or substituted phosphorus. In one embodiment, R¹ is an aliphatic moiety, a heteroaliphatic moiety, an aromatic moiety, or a heteroaromatic moiety, and in another embodiment is part of an aliphatic carbocycle, an aromatic carbocycle, a heterocyclic moiety, or a heteroaromatic moiety. The M, in the general metal complex 1, comprises one or more metals selected from a Group 4 element.

In some embodiments of the metal complex of the general metal complex 1, the Ar¹ and Ar³ are substituted with 1-4 hydrogens, 1-4 aromatic moieties, 1-4 aliphatic moieties, 1-4 substituted heteroatoms, or 1-4 halide moieties, and Ar² is substituted with 1-3 hydrogens, 1-3 aromatic moieties, 1-3 aliphatic moieties, 1-3 substituted heteroatoms, or 1-3 halide moieties.

In some embodiments, Zₙ of the general metal complex 1, includes 1-4 independently selected moieties that are either identical or different from one another, and the Zₙ moieties form covalent bonds, dative bonds, ionic bonds, or combinations thereof with M.

In more specific embodiments, the general metal complex 1 has the following metal complex structure: 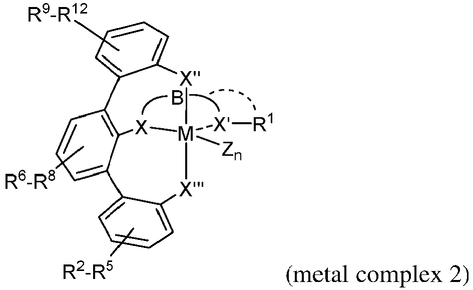 which is synthesized from a terphenyl ligand comprising the following structure:

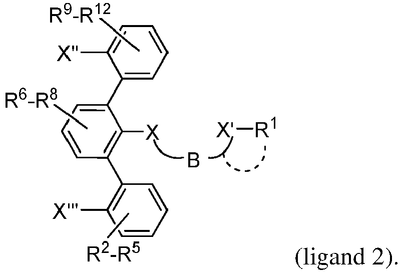

In metal complex 2 and ligand 2, the R²-R¹² are independently selected from aromatic moieties, aliphatic moieties, heteroaromatic moieties, heteroaliphatic moieties, heteroatoms, substituted heteroatoms, or halide moieties. In one embodiment, B has one to 15 atoms on shortest linkage path between X and X', not counting hydrogen atoms or side groups.

In some embodiments of the general metal complex 1 and metal complex 2, the metal complex has the following structure: 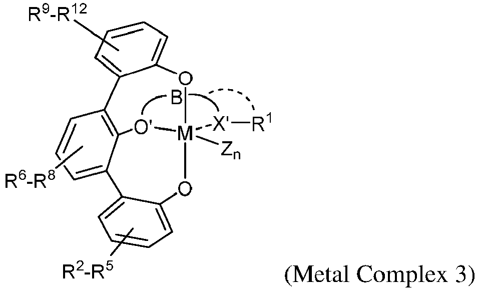 which is synthesized from a terphenyl ligand comprising the following structure:

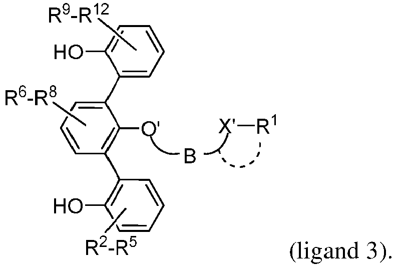

In further embodiments of the metal complex 3, the two O-M bonds are covalent; the O'-M bond is dative and the X'-M bond is dative or does not exist. In some embodiments of the metal complex 3 and the ligand 3, the R²-R¹² groups are independently selected from the same or different aromatic moieties, aliphatic moieties, heteroaromatic moieties, heteroaliphatic moieties, heteroatoms, substituted heteroatoms, or halide moieties. B has one to 10 atoms on shortest linkage path between O' and X', not counting H or side groups. In one embodiment of the metal complex 3, the Zₙ comprises 1-2 independent moieties that are either identical or different from one another, and wherein the Zₙ moieties form covalent bonds, dative bonds, ionic bonds, or combinations thereof with M.

In some embodiments, X' in general metal complex 1, metal complex 2, or metal complex 3 is part of an aliphatic carbocycle, an aromatic carbocycle, a heterocyclic moiety, or a heteroaromatic moiety.

The M of general metal complex 1, metal complex 2, or metal complex 3 may comprise transition metals, such as lanthanides, actinides, or Group 3 to Group 10 elements. In other embodiments, the M comprises a Group 4 element. In one embodiment, M comprises zirconium (Zr) or hafnium (Hf). The metal precursors may be monomeric, dimeric or higher orders thereof. Specific examples of suitable hafnium and zirconium precursors include, but are not limited to HfCl₄, Hf(CH₂Ph)₄, Hf(CH₂CMe₃)₄, Hf(CH₂SiMe₃)₄, Hf(CH₂Ph)₃Cl, Hf(CH₂CMe₃)₃Cl, Hf(CH₂SiMe₃)₃Cl, Hf(CH₂Ph)₂Cl2, Hf(CH₂CMe₃)₂Cl₂, Hf(CH₂SiMe₃)₂Cl₂, Hf(NMe₂)₄, Hf(NEt₂)₄, and Hf(N(SiMe₃)₂)₂Cl₂; ZrCl₄, Zr(CH₂Ph)₄, Zr(CH₂CMe₃)₄, Zr(CH₂SiMe₃)₄, Zr(CH₂Ph)₃Cl, Zr(CH₂CMe₃)₃Cl, Zr(CH2SiMe₃)₃Cl, Zr(CH₂Ph)₂Cl₂, Zr(CH₂CMe₃)₂Cl₂, Zr(CH₂SiMe₃)₂Cl₂, Zr(NMe₂)₄, Zr(NEt₂)₄, Zr(NMe₂)₂Cl₂, Zr(NEt₂)₂Cl₂, and Zr(N(SiMe3)₂)₂Cl₂. In the previous list, the "Me" stands for methyl, "Et" stands for ethyl, "Ph" stands for phenyl, and "THF" stands for tetrahydrofuran. Lewis base adducts of these examples are also suitable as metal precursors, for example, ethers, amines, thioethers, phosphines and the like are suitable as Lewis bases. Specific examples include HfCl₄(THF)₂, HfCl₄(SMe₂)₂ and Hf(CH₂Ph)₂Cl₂(OEt₂). Activated metal precursors may be ionic or zwitterionic compounds, such as (M(CH₂Ph)₃⁺)(B(C₆F₅)₄⁻) or (M(CH₂Ph)₃⁺) (PhCH₂B(C₆F₅)₃⁻) where M is defined above as comprising Hf or Zr.

In some embodiments, B in the metal complexes 1, 2 and 3 and the ligand 1, 2, and 3 is part of an aliphatic carbocycle, an aromatic carbocycle, or a heterocycle that connects onto R¹. The following ligand, ligand 4, shows the bridge, B, in bold and is not a simple alkyl chain, but is a part of another moiety. As shown below R¹, surrounded by a box, is a fragment or part of the moiety containing B. X' is the nitrogen atom, also in that moiety. Ligand 4 is one embodiment that depicts how one moiety may incorporate different groups - X', R¹, and B - within that one group in the general ligand 1 structure.

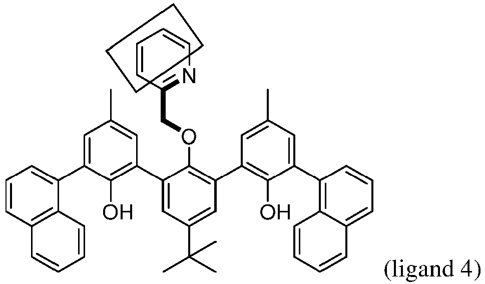

Ligand 4 also depicts the different R² to R¹² as six hydrogen, two naphthalene groups, two methyl groups, and a *tert*-butyl group.

In some embodiments, the ligands may comprise the following structures:

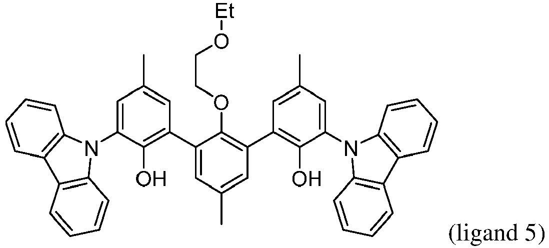

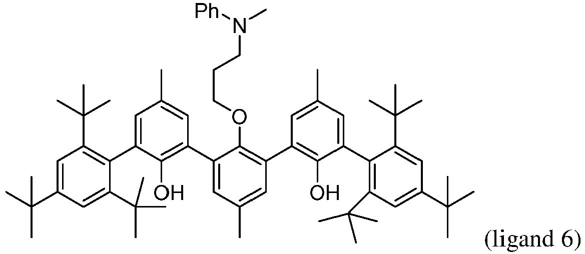

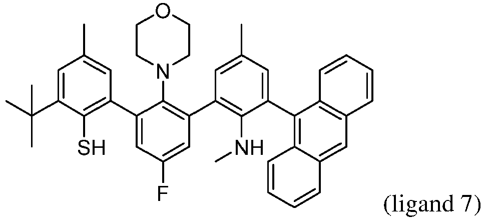

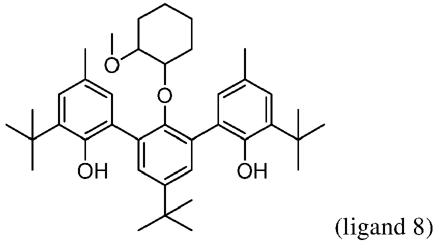

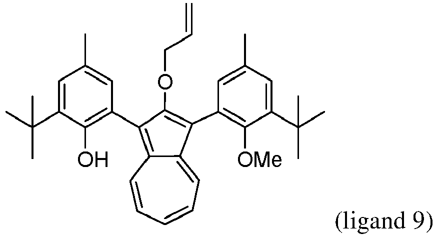

The Ar¹, Ar², and Ar³ comprise a type of aryl or heteroaryl group. In order for a group or molecule to be classified as "aromatic," it must: (1) have a planar cyclic structure; (2) every atom in the ring structure have an occupied *p* orbital; and (3) have an odd number of π-electron pairs represented by (4n + 2) where n is an integer including zero. For example, the Ar² group in ligand 9 is azulene, which has a fused aromatic 6 π-electron cyclopentadienyl anion and aromatic 6 π-electron tropylium cation. In order to achieve the stable aromatic sextet in both rings, one electron pair from the seven-membered ring is transferred to the five-membered ring. Reactivity studies confirm that the seven-membered ring is electrophilic and the five-membered ring is nucleophilic. Azulene has two rings fused together, but only accounts for one Ar group in the general ligand complex, while Ar¹ and Ar ³ are benzene rings that connect to the azulene group (Ar²) by a covalent bond. Azulene is one of the many possible aromatic groups that could be used in general metal complex 1 or general ligand 1.

Moreover, the X' in ligand 9 is a vinyl carbon substituent. In theory, and not excluding alternative possibilities, the electrons in the *p* orbital of the carbon double bond can coordinate with the metal to form a metal complex. This is comparable to how the lone pair of electrons in an oxygen or nitrogen atom interacts with the metal species.

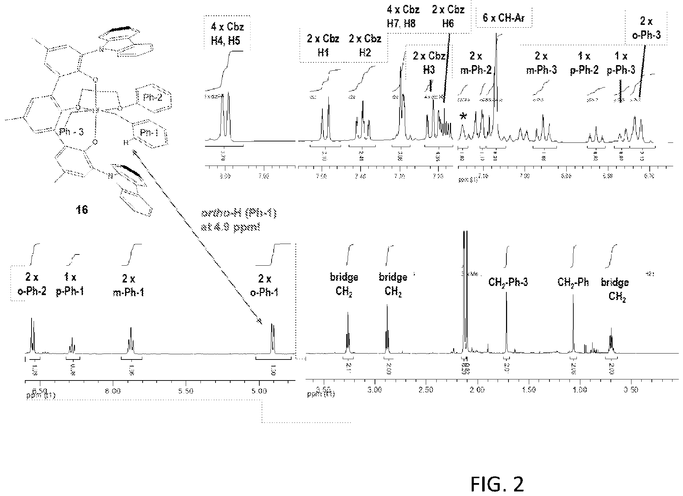

The general structure 1, metal complex 1, and metal complex 2 have a Cₛ-symmetrical meridional (mer) isomer. A plane of symmetry extends through the Ar², X, M, Xₙ, and the bridging component, B, thereby reflecting Ar₁ and Ar₃ into one another. Nuclear Magnetic Resonance (NMR) studies provide evidence that some embodiments of the metal complexes in this disclosure have a Cₛ-symmetry. When a molecule has a diastereotopic center, then the two protons on a secondary carbon would be in different chemical environments. If there is no plane of symmetry, then splitting patterns in the proton NMR (H-NMR) would be more complex than if a plane of symmetry bisected the secondary carbon. For example, the NMR results of Hf-L10 in the Example 7 and FIG. 2 shows two singlet signals, one for *CH*₂Ph₁ and one for *CH*₂Ph₂; both signals have an integration of two, which means both protons on the carbon atom are accounted for in one signal. This indicates that the protons on these carbon atoms are related by the plane of symmetry. If there was not a plane of symmetry, then the protons would be diastereotopic. On the NMR spectrum, there might be two doublets each having an integration of one in the place of one singlet. This effect results from the protons being in different chemical environments, which causes one proton on the carbon atom to split the magnetic resonance of the other proton, thereby creating separate and distinct proton signals.

Although, proton NMR (H-NMR) and carbon-13 NMR (C-NMR) indicates that there is a plane of symmetry as described in the preceding paragraph, NMR signals also indicate that the complex has a pseudo Cₛ-symmetry. The bridging arm, B, can move or flip outside of the plane of symmetry. The signals in the C-NMR and H-NMR indicate that the bridging arm oscillates above and below the plane of symmetry, thereby breaking the plane of symmetry. The break in the plane of symmetry causes the complex to have a pseudo Cₛ-symmetry.

Regardless of the pseudo symmetry, the Cₛ-symmetrical catalysts produce pseudo-tactic polymers, especially when propylene is the base monomer. Due to the structure and symmetry of the complex or catalyst, there are two active sites in the complex. The two active sites create polymers with low molecular weights, good adhesive properties, and reasonable octene incorporation.

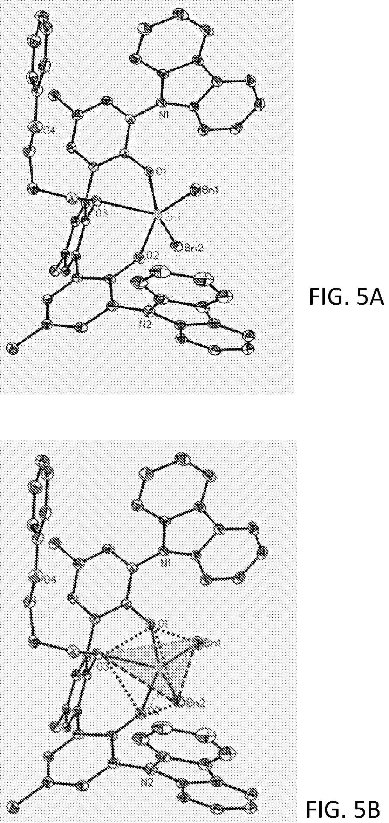

When there are two Zₙ groups in a metal complex as in FIGS. 5A-B, the crystal structure of the metal complex shows a distorted trigonal bipyramidal complex. The structures in FIGS. 5A-B show two crystal structure, both structures depict the crystal structure of L10-Zr. Both structures are a crystal structure of a distorted trigonal bipyramidal complex, and outlined on the second structure is the geometrical configuration. X is represented by O3, Zₙ¹ is represented by Bn1 and Zₙ² is represented by Bn2. These three groups form the center triangle and X" represent by O1 and X'" represented by O3 are the two apices. The distortion in the crystal structure results from the X' being detached from the metal center. Theoretically, if the second oxygen were bound to the metal center, the expected crystal structure would have an octahedral or distorted octahedral geometry.

Schemes 1 and 2, as follows, illustrate a possible synthetic scheme for achieving one possible ligand as described in this disclosure. The ligands 5 and 10, depending on the R group, are tertadentate terphenol ligands. Another advantage to the catalysts in this disclosure is the ease of synthesis. As discussed in the following paragraphs, the starting materials are commercially available, which decreases the number of synthetic steps. Additional examples will be provided in the Example section to follow.

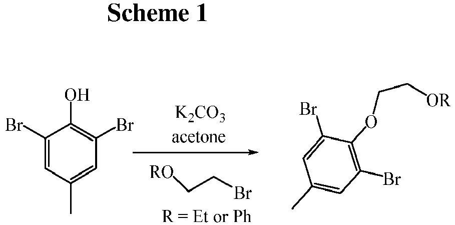

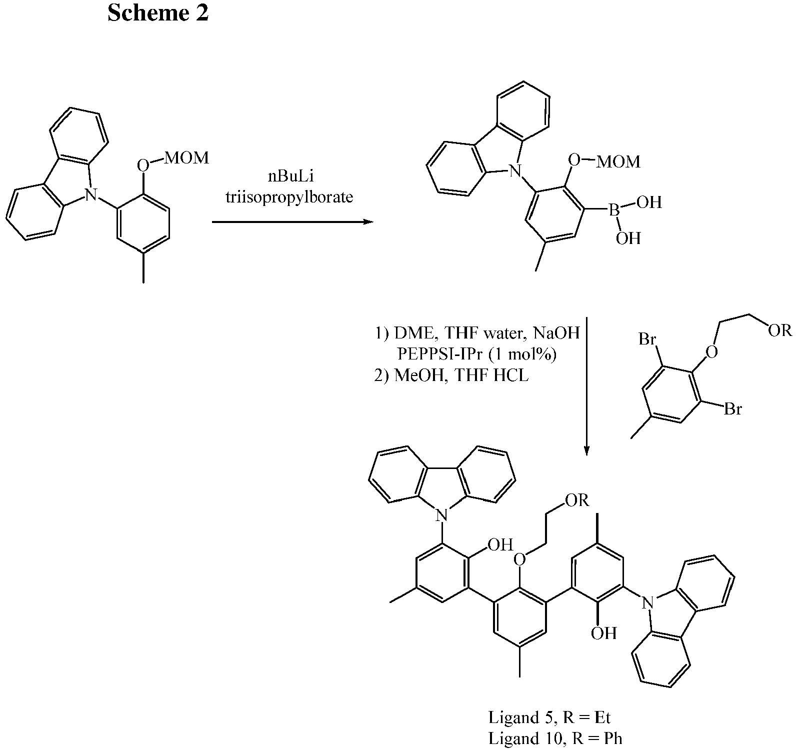

In some embodiments, the ligand of general metal ligand complex (I), which can be synthesized from Schemes 1 and 2, or another similar scheme, is any one of the following:

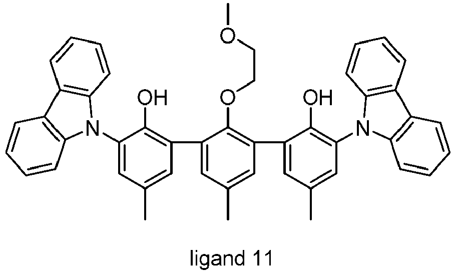

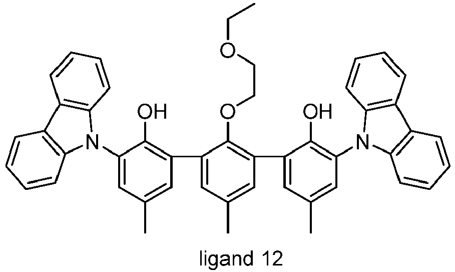

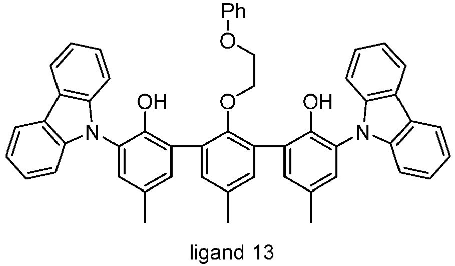

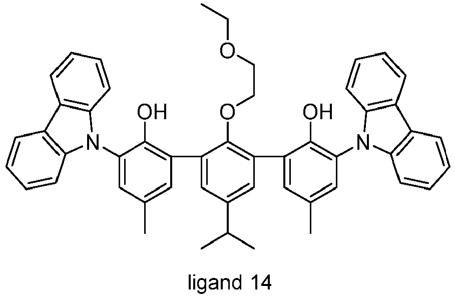

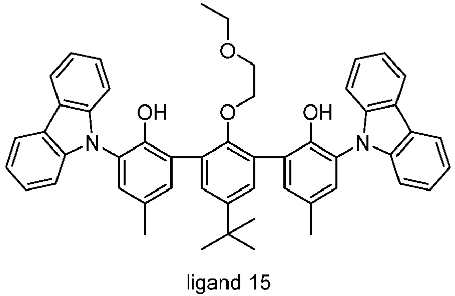

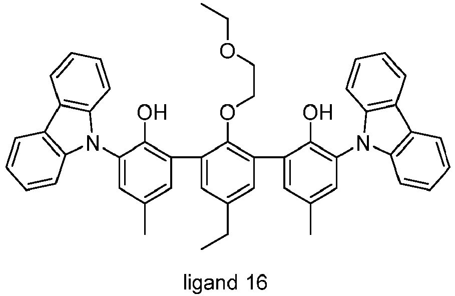

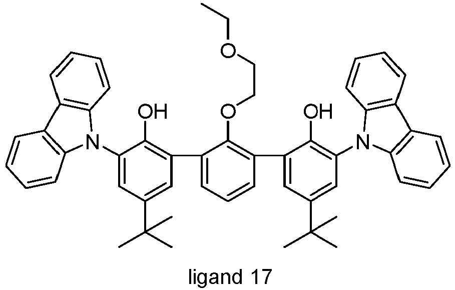

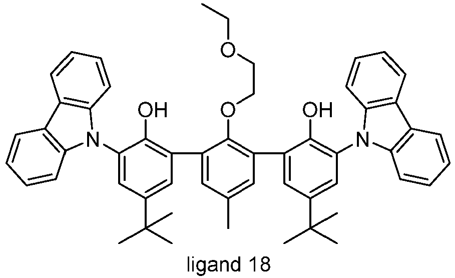

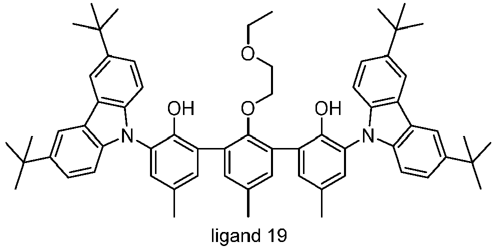

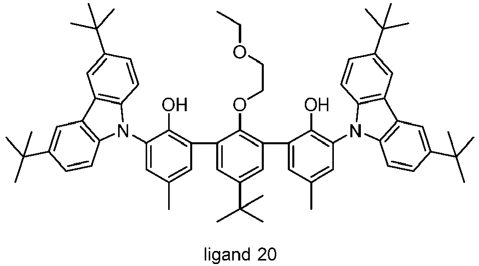

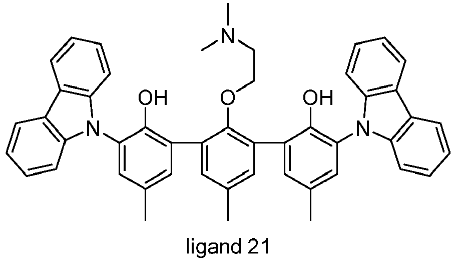

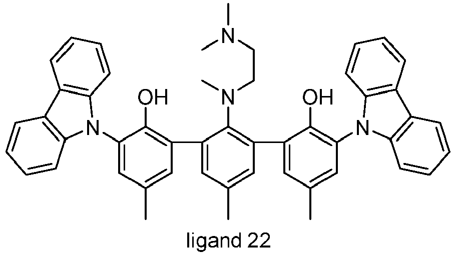

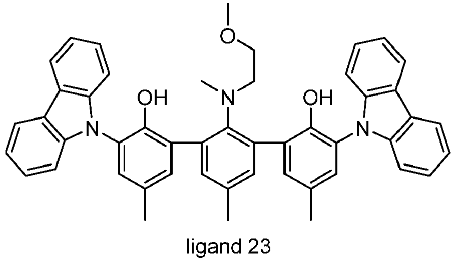

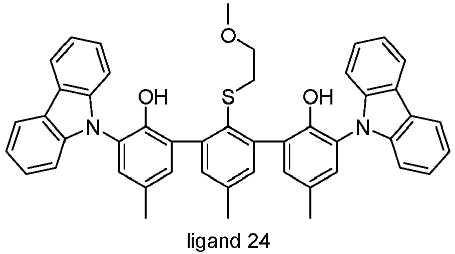

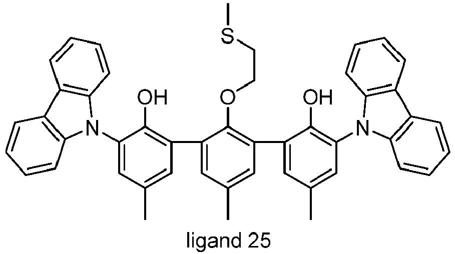

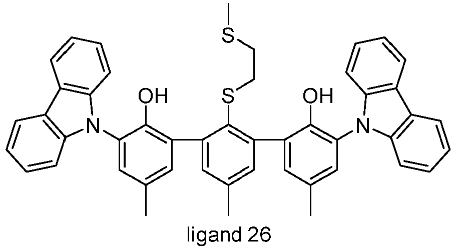

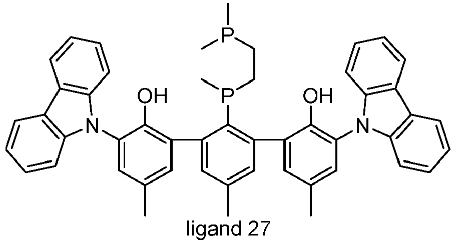

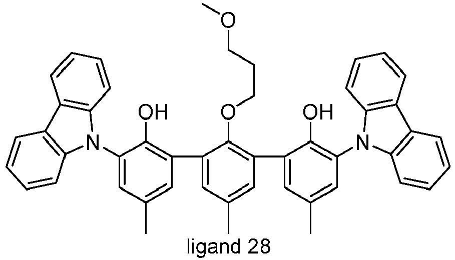

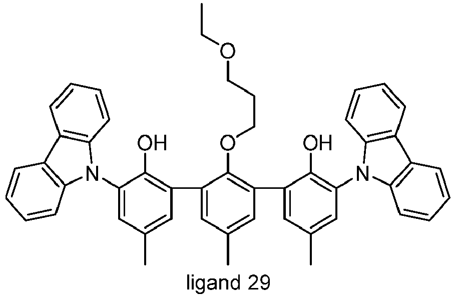

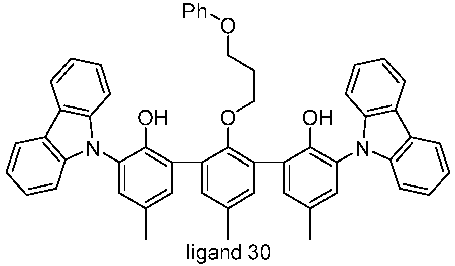

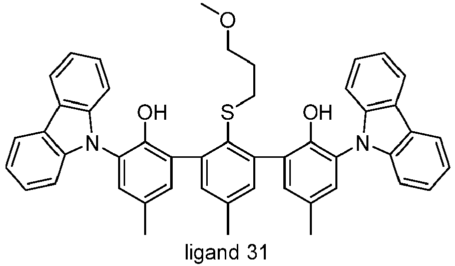

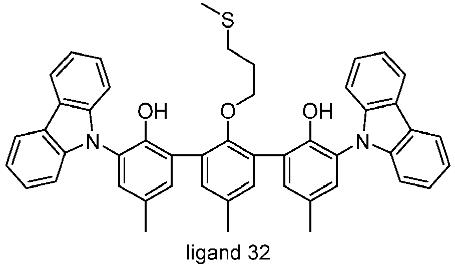

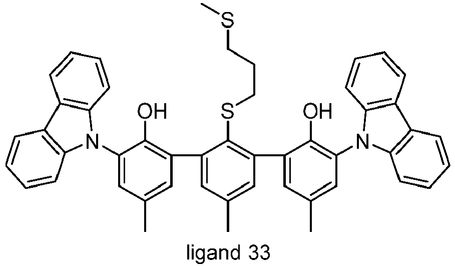

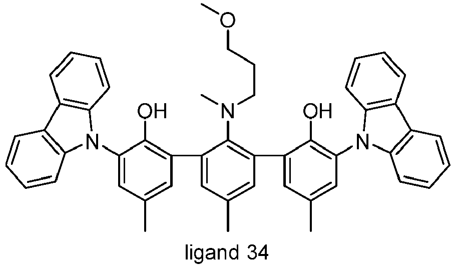

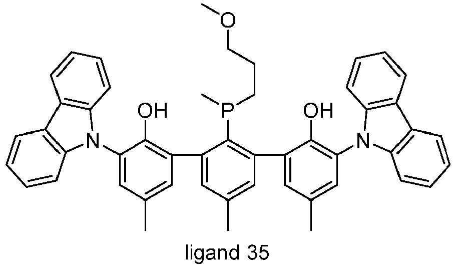

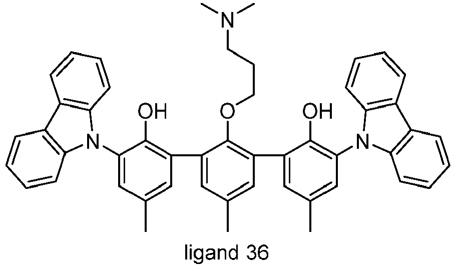

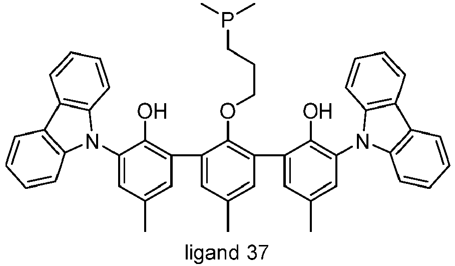

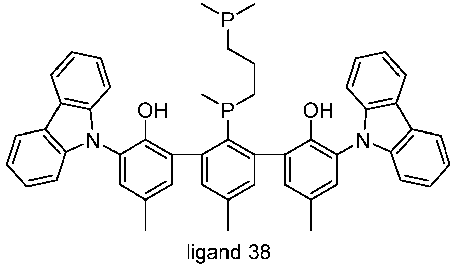

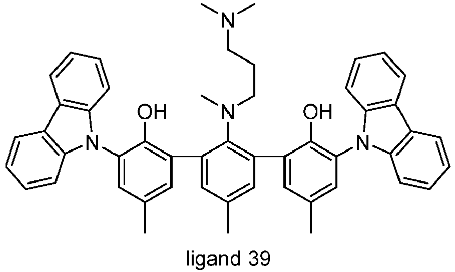

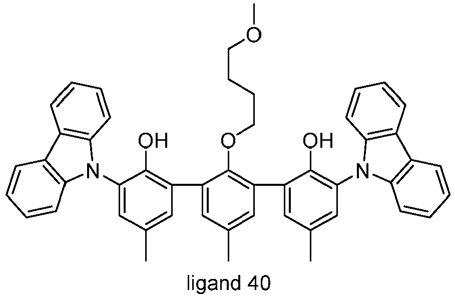

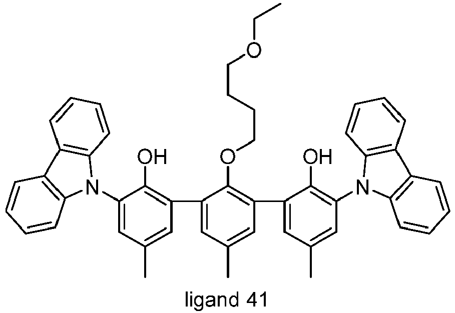

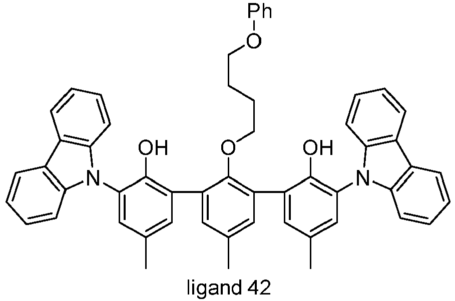

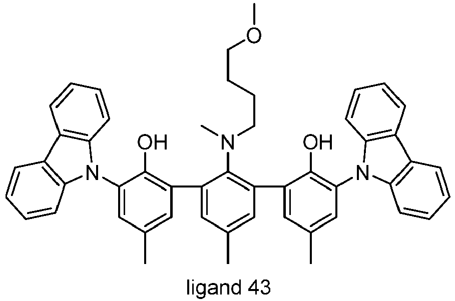

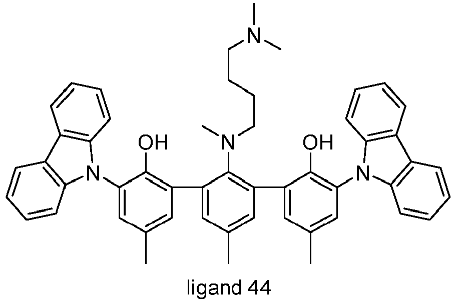

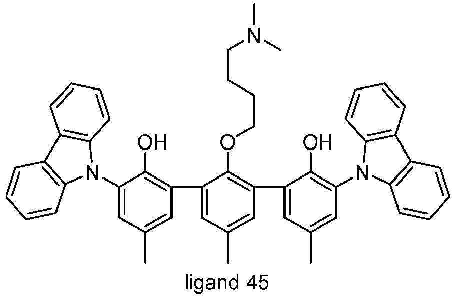

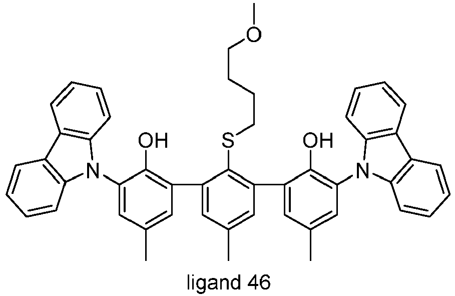

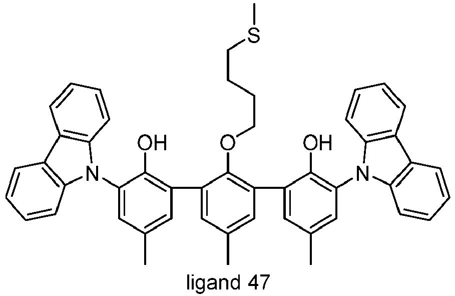

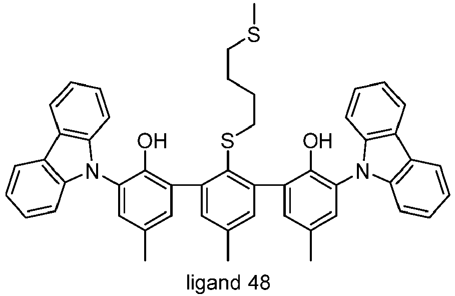

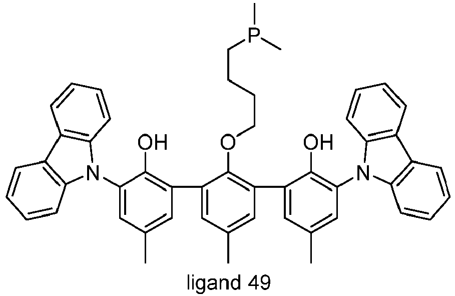

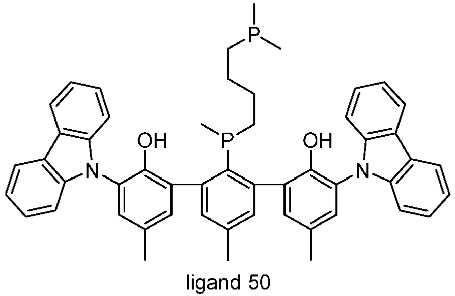

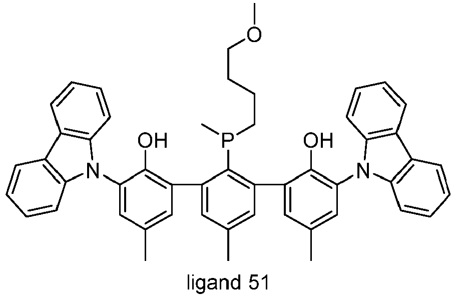

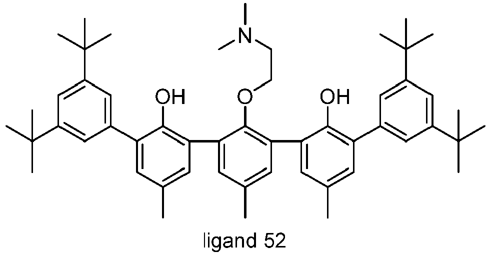

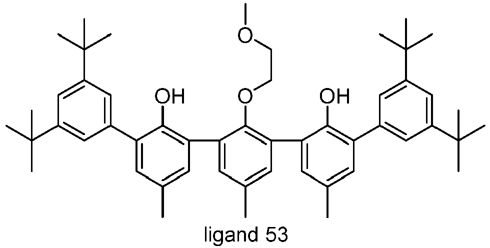

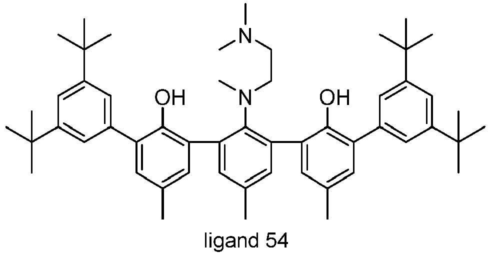

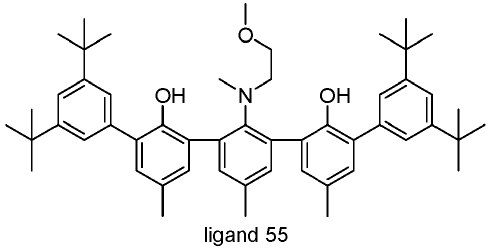

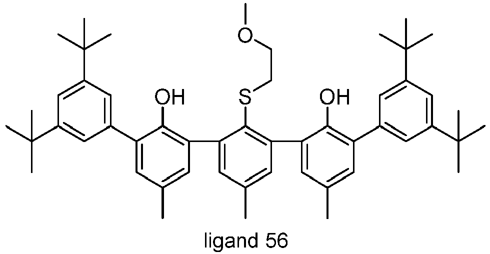

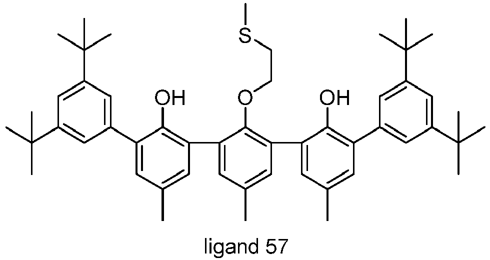

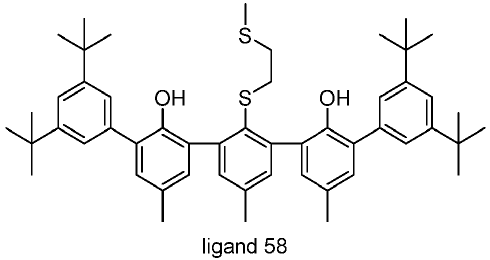

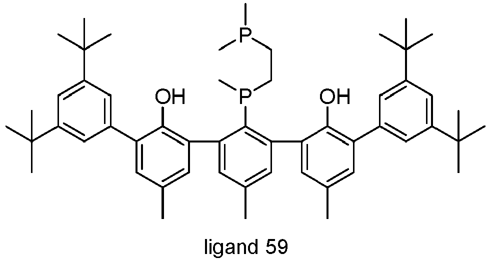

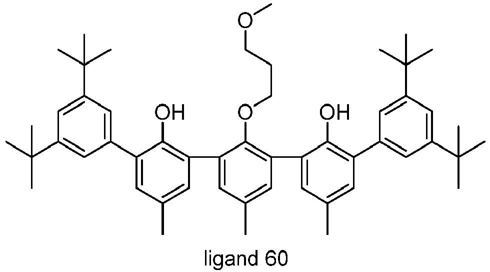

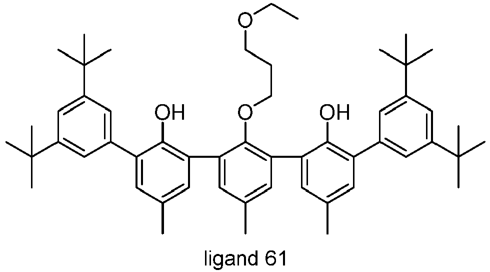

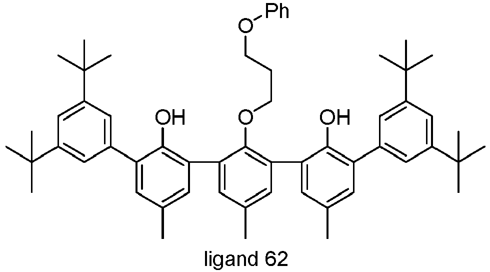

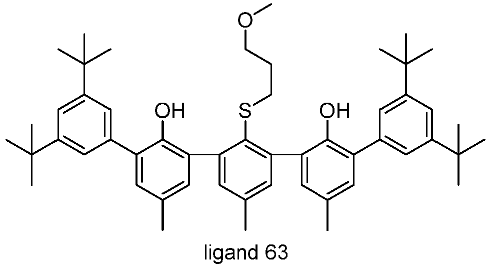

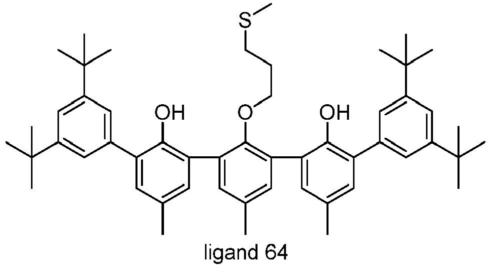

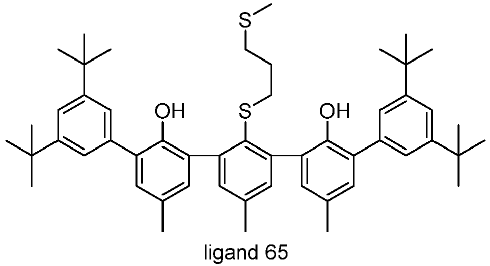

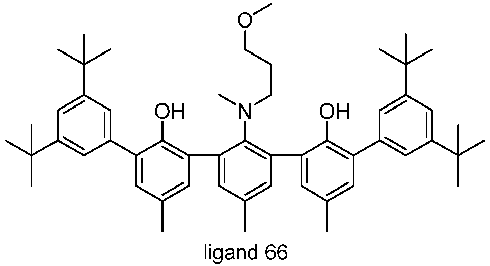

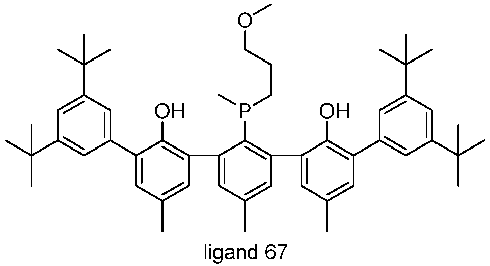

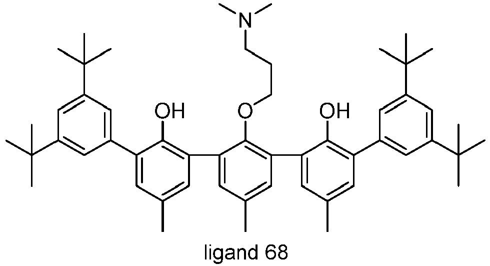

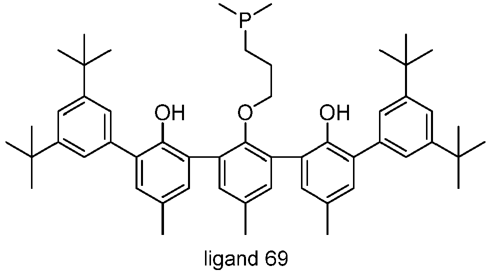

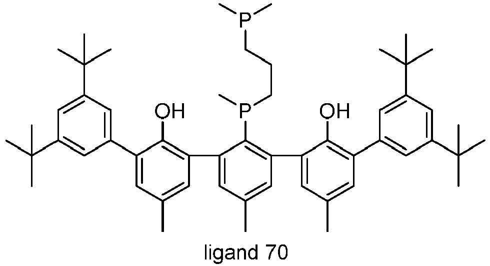

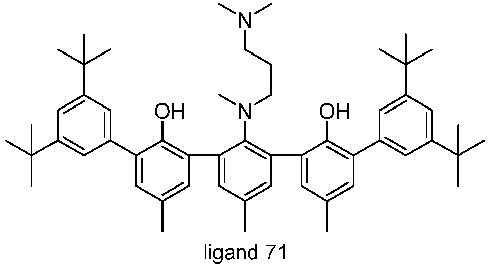

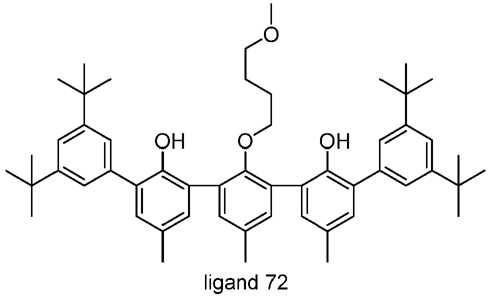

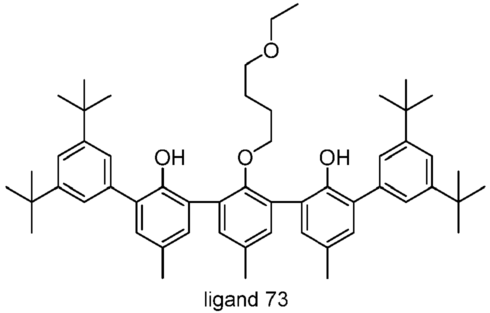

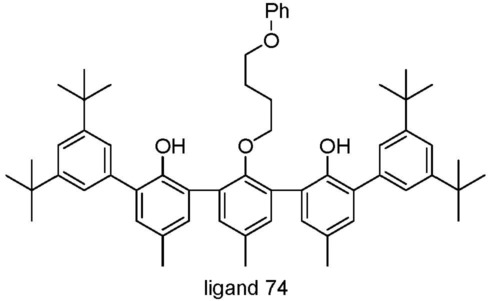

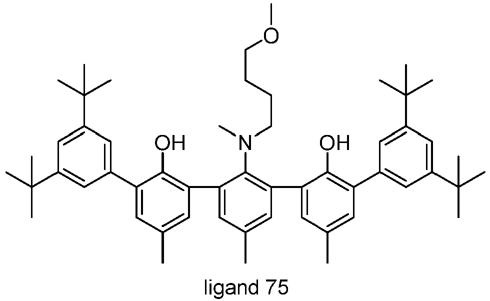

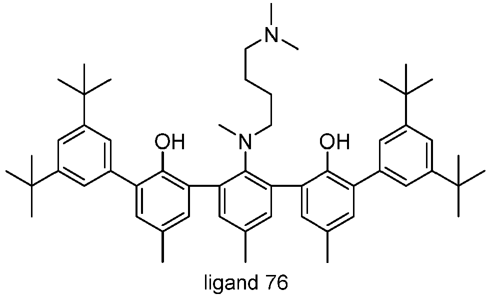

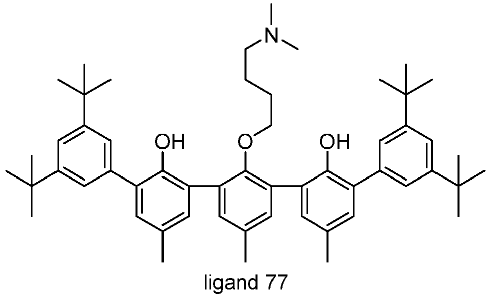

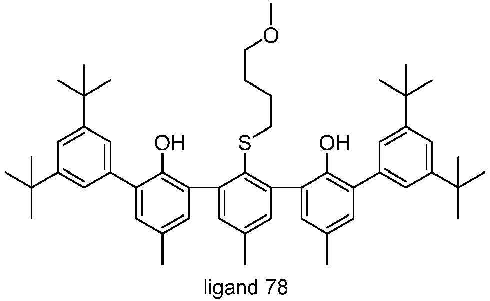

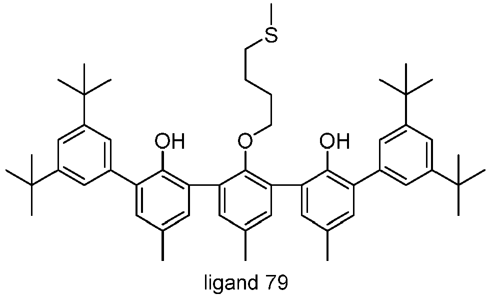

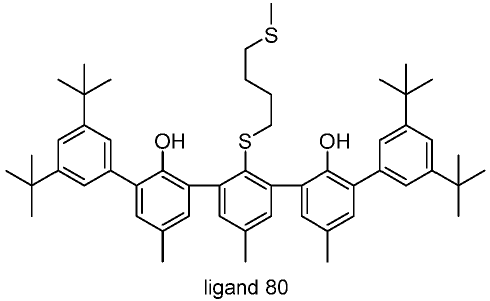

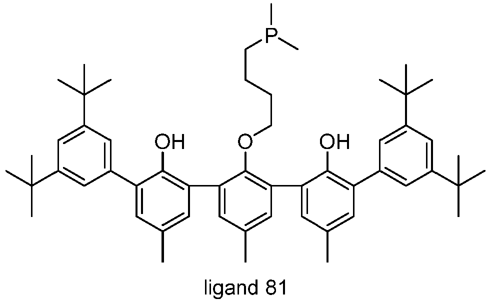

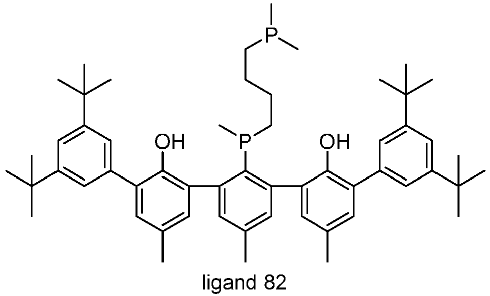

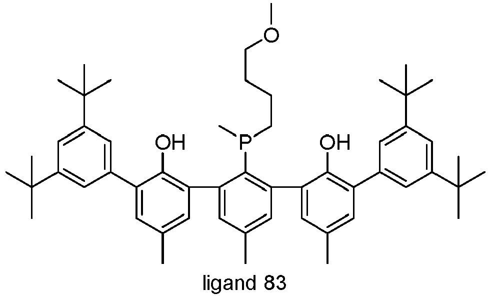

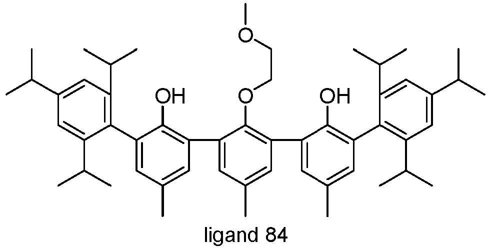

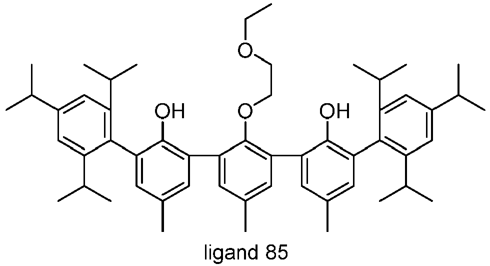

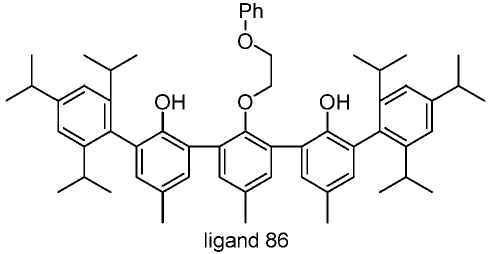

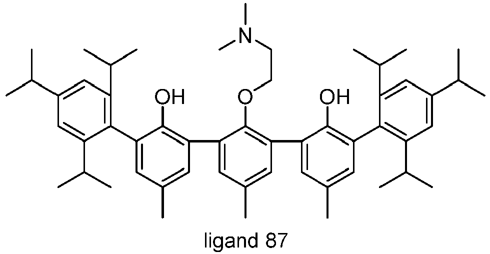

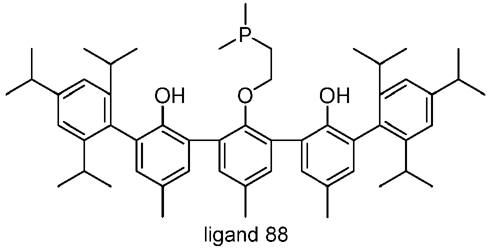

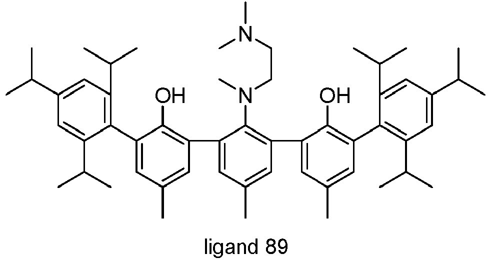

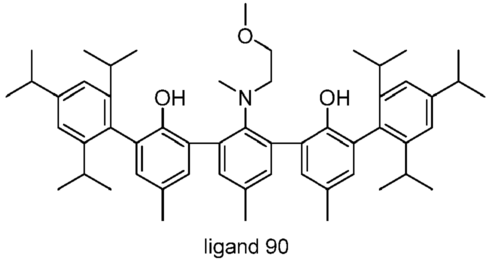

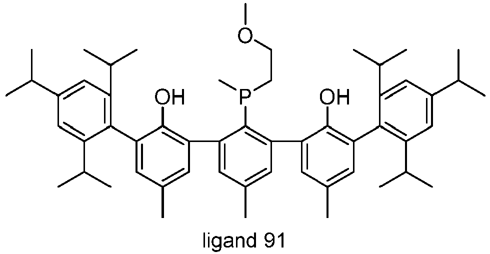

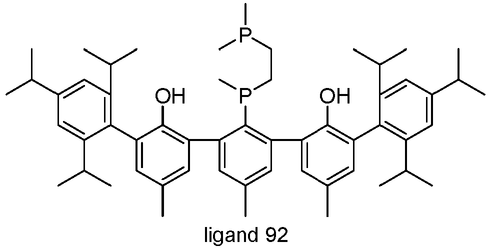

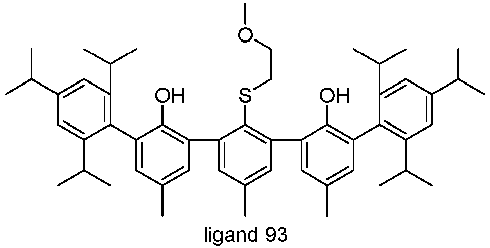

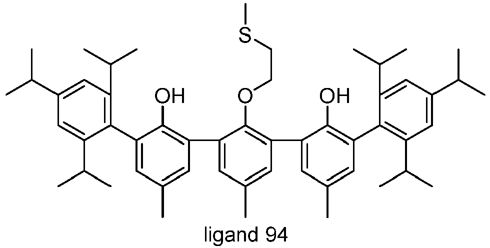

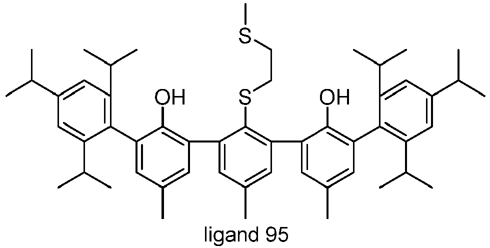

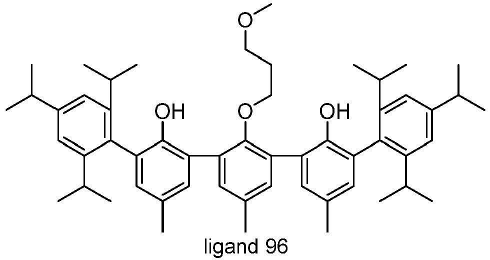

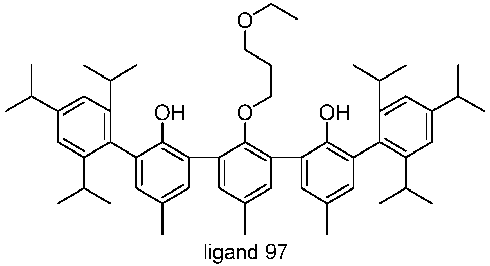

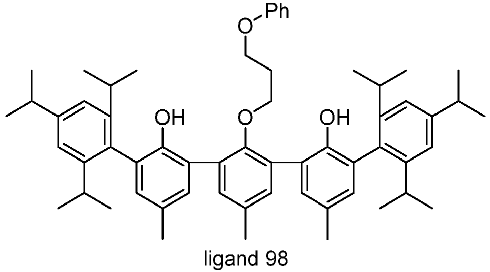

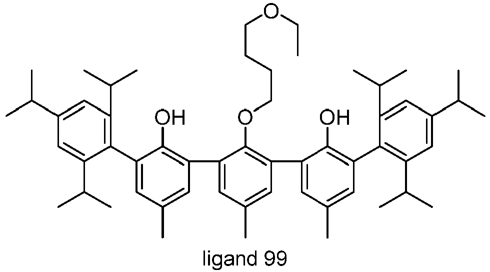

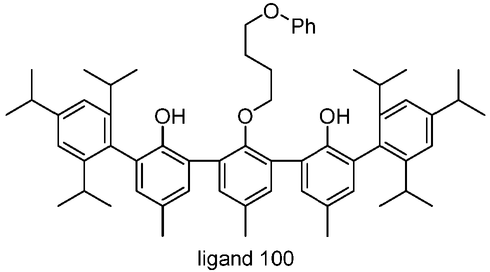

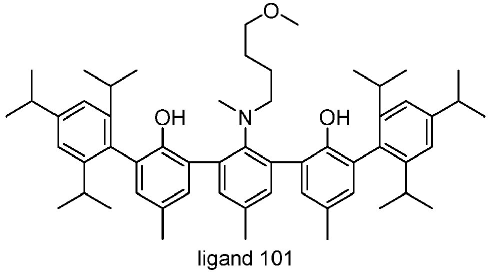

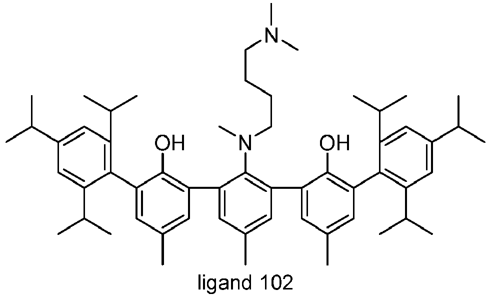

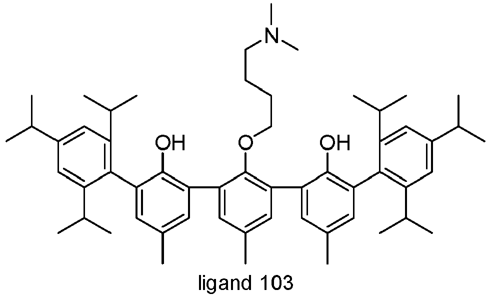

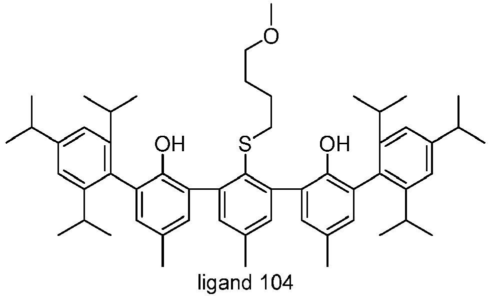

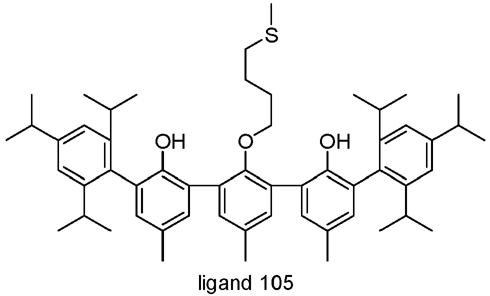

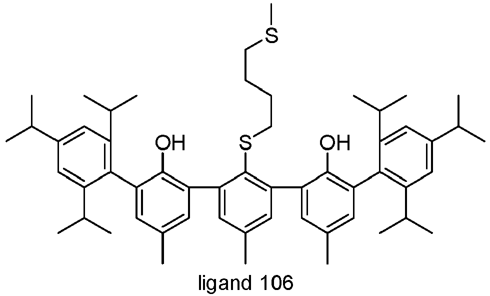

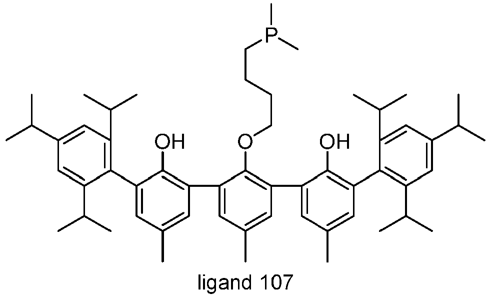

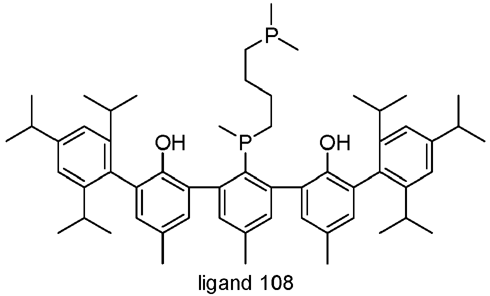

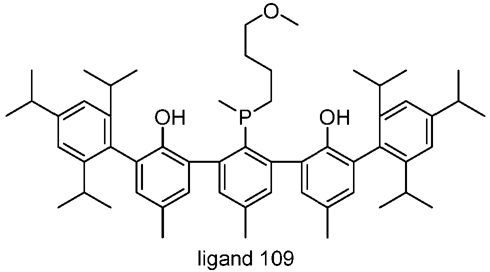

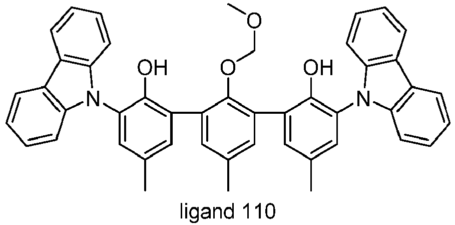

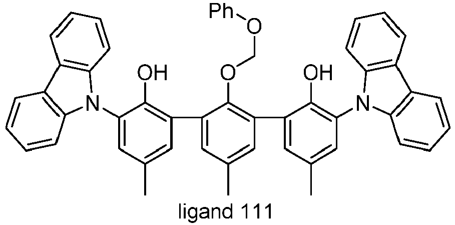

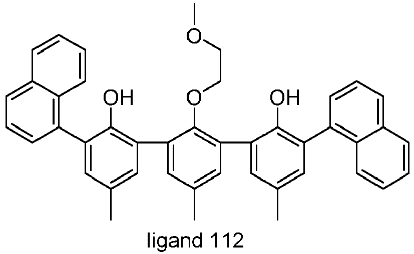

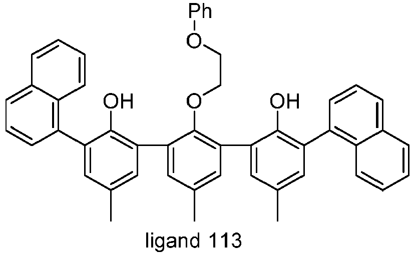

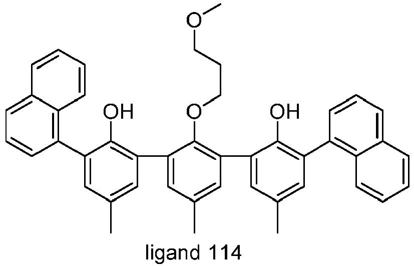

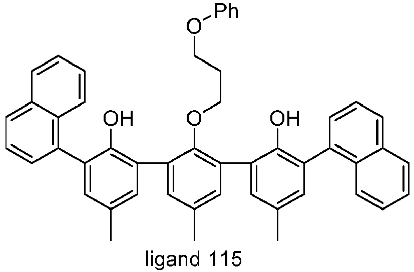

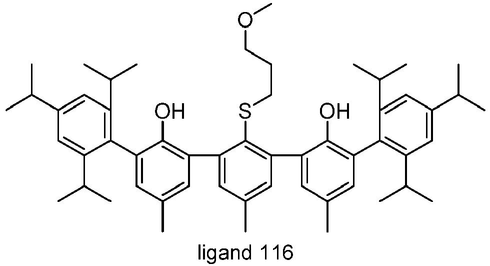

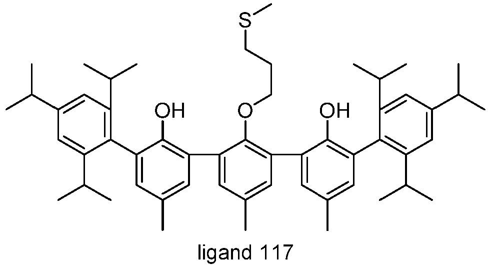

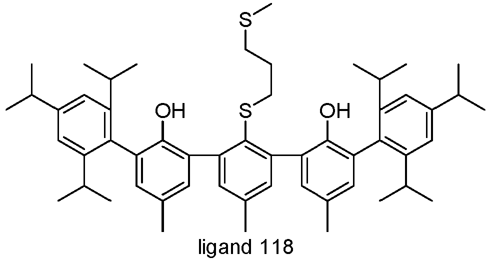

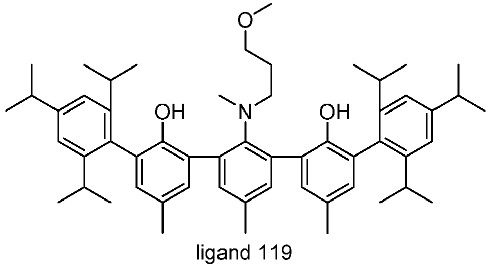

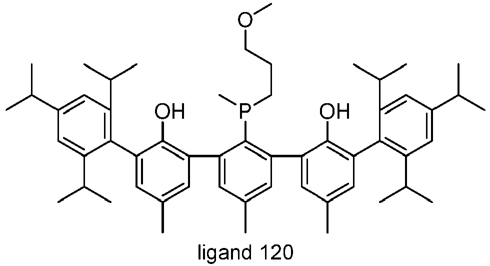

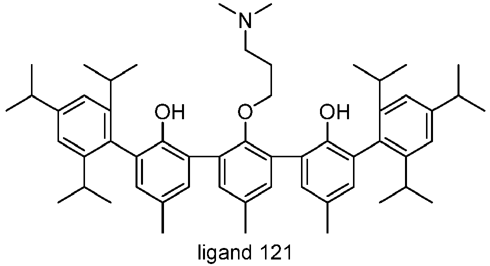

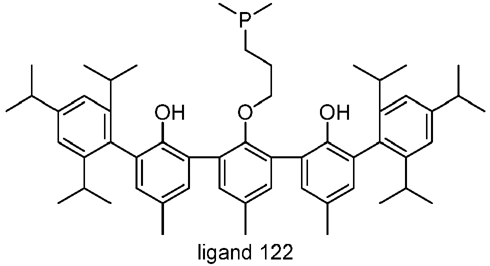

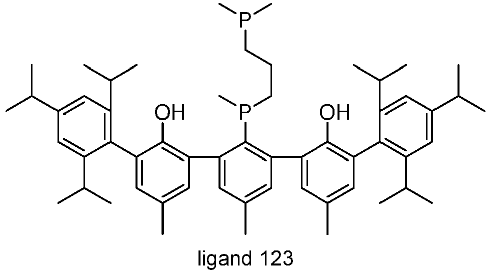

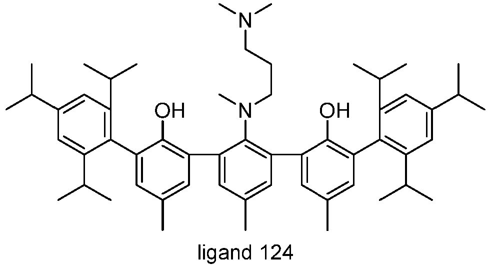

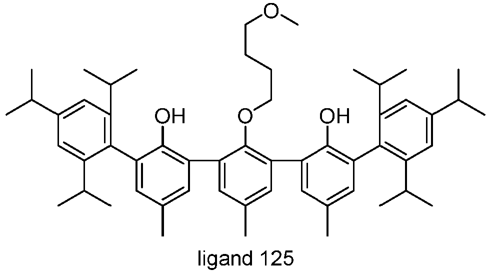

The metal complexes, formed from the ligands, can have multiple reaction sites, while some have single site reaction. Some of the catalyst complexes result in a bimodal polymer. Additionally, the present catalyst complex may provide good secondary α-olefins (e.g. 1-octene) incorporation. For example and not by way of limitation, the catalyst may yield a polymer which incorporates at least 20 weight percent (wt %) of the secondary alpha olefin. From an application standpoint, these catalyst complexes may also yield polymers with good adhesive properties. While some complexes yield only one or two of the three aforementioned results. These ligands create versatile catalysts, but an added benefit is that these catalysts are easily synthesized. Scheme 1 and Scheme 2 show that different functional groups and bridge, B, can be interchanged by changing one reagent.

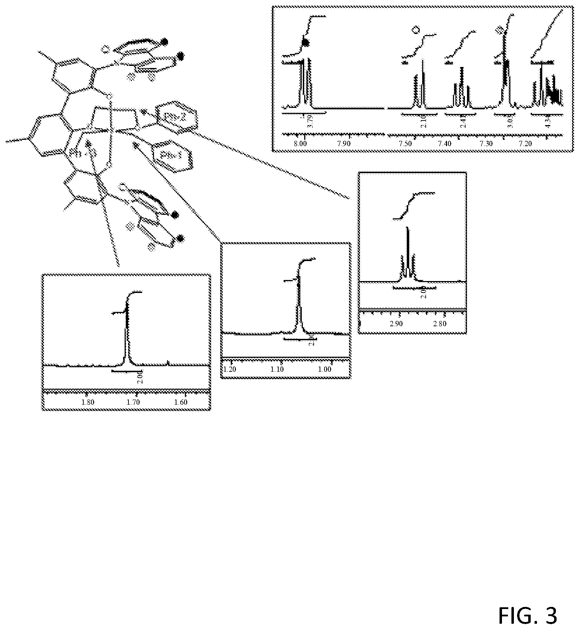

In FIG. 3, there are fragments of an H-NMR of bis-ether hafnium metal complex synthesized from ligand 10. There are three proton signals that indicate that the metal complex has an apparent Cₛ-symmetry on the NMR timescale. The three signals designated by three circles - white, gray and black - on the carbazole moiety show that the carbazole moiety cannot freely spin. Otherwise, if the carbazole moiety is environmentally unrestricted and is freely able to spin, then the signal marked by the white circle would be identical to the signal marked by the gray circle. However, the gray circle and the white circle indicate different signals, which show the protons are in different chemical environments.

The polymers synthesized with the catalytic complexes of the present disclosure can be homopolymers of C₂-C₂₀ alpha-olefins, such as ethylene, propylene, or 4-methyl-1-pentene, or they may be interpolymers of ethylene or propylene with at least one or more alpha-olefins and/or C₂-C₂₀ acetylenically unsaturated monomers and/or C₄-C₁₈ diolefins. They may also be interpolymers of ethylene with at least one of the above C₃-C₂₀ alpha-olefins, diolefins and/or acetylenically unsaturated monomers in combination with other unsaturated monomers. For example, in one embodiment ethylene or a mixture of ethylene and from about 0.1 to about 20 wt %, for example, from about 0.1 to about 15 wt %, or in the alternative, from about 0.1 to about 10 wt %; or in the alternative, from 0.1 to about 5 weight percent of 1-hexene, 1-octene, or a similar higher α-olefin, based on total monomer in the final copolymer, may be successfully polymerized using the disclosed catalyst composition.

In the polymerization process employing the aforementioned catalytic reaction product, polymerization is effected by adding a catalytic amount of the disclosed catalyst composition to a polymerization reactor containing the selected α-olefin monomer or monomers, or vice versa. The polymerization reactor is maintained at temperatures in the range from 150 degrees Celsius (°C) to 300 °C, or at solution polymerization temperatures, such as from 150 °C to 250 °C, for a residence time, in certain non-limiting embodiments, ranging from 5 minutes to 20 minutes. Longer or shorter residence times may alternatively be employed. Generally, the polymerization reaction is carried out in the absence of moisture and oxygen. The anaerobic atmosphere is maintained in the presence of a catalytic amount of the catalytic reaction product that is typically within the range from 0.0001 to about 0.01 milligram-atoms metal catalyst per liter of diluent. However, catalyst concentration depends upon polymerization conditions such as temperature, pressure, solvent and the presence of catalyst poisons and that the foregoing range is given for illustrative purposes of one particular but non-limiting embodiment only. For example, pressures may be relatively low, such as from 150 to 3,000 psig (1.0 to 20.7 MPa), from 250 to 1,000 psig (1.7 to 6.9 MPa), or from 450 to 800 psig (3.1 to 5.5 MPa). However, polymerization within the scope of the invention can occur at pressures from atmospheric up to pressures determined by the capabilities of the polymerization equipment.

Generally in the polymerization process, a carrier, which may be an inert organic diluent or solvent or excess monomer, is employed and oversaturation of the solvent with polymer is avoided. If such saturation occurs before the catalyst becomes depleted, the catalyst may not reach its full efficiency. In particular embodiments, the amount of polymer in the carrier does not exceed 30 percent, based on the total weight of the reaction mixture. Stirring the polymerization components enhances the uniformity of the polymerization by controlling and maintaining an invariable temperature throughout the polymerization zone. For example, in the case of relatively more rapid reactions with relatively active catalysts, means may be provided for refluxing monomer and diluent, if diluent is included, thereby removing some of the heat of reaction. In any event, adequate means should be provided for dissipating the exothermic heat of polymerization. Thus, polymerization may be effected in a batch manner, or in a continuous manner, such as, for example, by passing the reaction mixture through an elongated reaction tube which is contacted externally with suitable cooling medium to maintain the desired reaction temperature, or by passing the reaction mixture through an equilibrium overflow reactor or a series of the same.

In order to enhance catalyst efficiency in the polymerization of ethylene, maintaining a certain ethylene concentration in the diluents ensures reactor stability and optimizes catalyst efficiency. In some embodiments this may include a ratio of solvent to ethylene ranging from 1:2 to 1:8 or 1:3 to 1:5. To achieve this ratio, when an excess of ethylene is fed into the system, a portion of the ethylene can be vented.

Hydrogen is often employed in the practice of this polymerization system, for the purpose of lowering the molecular weight of the resultant polymer, if a lower molecular weight is desirable. It may be beneficial to employ hydrogen in the polymerization mixture in concentrations ranging preferably from 0.001 to 1 mole per mole of monomer. The larger amounts of hydrogen within this range generally produces lower molecular weight polymer. Those skilled in the art known that hydrogen may be added to the polymerization vessel either with a monomer stream, or separately, before, during or after addition of the monomer to the polymerization vessel. However, hydrogen should be added either before or during addition of the catalyst, in the range of from 200,000 to 3 million grams of polymer per gram of metal in the catalyst.

The resulting polymer may be recovered from the polymerization mixture by driving off unreacted monomer and diluent, where such is employed. No further removal of impurities is required.

The procatalyst comprising the metal-ligand complex of general metal complex 1 is rendered catalytically active by contacting it to, or combining it with, the activating co-catalyst or by using an activating technique such as those that are known in the art for use with metal-based olefin polymerization reactions. Suitable activating co-catalysts for use herein include alkyl aluminums; polymeric or oligomeric alumoxanes (also known as aluminoxanes); neutral Lewis acids; and non-polymeric, non-coordinating, ion-forming compounds (including the use of such compounds under oxidizing conditions). A suitable activating technique is bulk electrolysis. Combinations of one or more of the foregoing activating co-catalysts and techniques are also contemplated. The term "alkyl aluminum" means a monoalkyl aluminum dihydride or monoalkylaluminum dihalide, a dialkyl aluminum hydride or dialkyl aluminum halide, or a trialkylaluminum. Aluminoxanes and their preparations are known at, for example, United States Patent Number (USPN) U.S. Pat. No. 6,103,657. Examples of preferred polymeric or oligomeric alumoxanes are methylalumoxane, triisobutylaluminum-modified methylalumoxane, and isobutylalumoxane.

Exemplary Lewis acid activating co-catalysts are Group 13 metal compounds containing from 1 to 3 hydrocarbyl substituents as described herein. In some embodiments, exemplary Group 13 metal compounds are tri(hydrocarbyl)-substituted-aluminum or tri(hydrocarbyl)-boron compounds. In some other embodiments, exemplary Group 13 metal compounds are tri(hydrocarbyl)-substituted-aluminum or tri(hydrocarbyl)-boron compounds are tri((C₁-C₁₀)alkyl)aluminum or tri((C₆-C₁₈)aryl)boron compounds and halogenated (including perhalogenated) derivatives thereof. In some other embodiments, exemplary Group 13 metal compounds are tris(fluoro-substituted phenyl)boranes, in other embodiments, tris(pentafluorophenyl)borane. In some embodiments, the activating co-catalyst is a tris((C₁-C₂₀)hydrocarbyl) borate (e.g., trityl tetrafluoroborate) or a tri((C₁-C₂₀)hydrocarbyl)ammonium tetra((C₁-C₂₀)hydrocarbyl)borane (e.g., bis(octadecyl)methylammonium tetrakis(pentafluorophenyl)borane). As used herein, the term "ammonium" means a nitrogen cation that is a ((C₁-C₂₀)hydrocarbyl)₄N⁺, a ((C₁-C₂₀)hydrocarbyl)₃N(H)⁺, a ((C₁-C₂₀)hydrocarbyl)₂N(H)₂⁺, (C₁-C₂₀)hydrocarbylN(H)₃⁺, or N(H)₄⁺, wherein each (C₁-C₂₀)hydrocarbyl may be the same or different.

Exemplary combinations of neutral Lewis acid activating co-catalysts include mixtures comprising a combination of a tri((C₁-C₄)alkyl)aluminum and a halogenated tri((C₆-C₁₈)aryl)boron compound, especially a tris(pentafluorophenyl)borane. Other exemplary embodiments are combinations of such neutral Lewis acid mixtures with a polymeric or oligomeric alumoxane, and combinations of a single neutral Lewis acid, especially tris(pentafluorophenyl)borane with a polymeric or oligomeric alumoxane. Exemplary embodiments ratios of numbers of moles of (metal-ligand complex):(tris(pentafluoro-phenylborane): (alumoxane) [e.g., (Group 4 metal-ligand complex):(tris(pentafluoro-phenylborane):(alumoxane)] are from 1:1:1 to 1:10:30, other exemplary embodiments are from 1:1:1.5 to 1:5:10.

Many activating co-catalysts and activating techniques have been previously taught with respect to different metal-ligand complexes in the following USPNs: U.S. Pat. No. 5,064,802; U.S. Pat. No. 5,153,157; U.S. Pat. No. 5,296,433; U.S. Pat. No. 5,321,106; U.S. Pat. No. 5,350,723; U.S. Pat. No. 5,425,872; U.S. Pat. No. 5,625,087; U.S. Pat. No. 5,721,185; U.S. Pat. No. 5,783,512; U.S. Pat. No. 5,883,204; U.S. Pat. No. 5,919,983; U.S. Pat. No. 6,696,379; and U.S. Pat. No. 7,163,907. Examples of suitable hydrocarbyloxides are disclosed in U.S. Pat. No. 5,296,433. Examples of suitable Bronsted acid salts for addition polymerization catalysts are disclosed in U.S. Pat. No. 5,064,802; U.S. Pat. No. 5,919,983; U.S. Pat. No. 5,783,512. Examples of suitable salts of a cationic oxidizing agent and a non-coordinating, compatible anion as activating co-catalysts for addition polymerization catalysts are disclosed in U.S. Pat. No. 5,321,106. Examples of suitable carbenium salts as activating co-catalysts for addition polymerization catalysts are disclosed in U.S. Pat. No. 5,350,723. Examples of suitable silylium salts as activating co-catalysts for addition polymerization catalysts are disclosed in U.S. Pat. No. 5,625,087. Examples of suitable complexes of alcohols, mercaptans, silanols, and oximes with tris(pentafluorophenyl)borane are disclosed in U.S. Pat. No. 5,296,433. Some of these catalysts are also described in a portion of U.S. Pat. No. 6,515,155 B1 beginning at column 50, at line 39, and going through column 56, at line 55.

In some embodiments, the procatalyst comprising the metal-ligand complex of general metal complex 1 may be activated to form an active catalyst composition by combination with one or more cocatalyst. A non-limiting list of possible cocatalysts include: strong Lewis acids; compatible, noncoordinating, ion forming compounds, such as bis(hydrogenated tallow alkyl)methyl ammonium and tetrakis(pentafluorophenyl)borate(1-) amine; a cation forming cocatalyst; polymeric or oligomeric aluminoxanes, especially methyl aluminoxane and modified methyl aluminoxane (MMAO); orgoaluminum compounds, such as triethyl aluminum (TEA); and any combinations thereof.

In some embodiments, one or more of the foregoing activating co-catalysts are used in combination with each other. An especially preferred combination is a mixture of a tri((C₁-C₄)hydrocarbyl)aluminum, tri((C₁-C₄)hydrocarbyl)borane, or an ammonium borate with an oligomeric or polymeric alumoxane compound.

The ratio of total number of moles of one or more metal-ligand complexes of general metal complex 1 to total number of moles of one or more of the activating co-catalysts is from 1:10,000 to 100:1. In some embodiments, the ratio is at least 1:5000, in some other embodiments, at least 1:1000; and 10:1 or less, and in some other embodiments, 1:1 or less. When an alumoxane alone is used as the activating co-catalyst, preferably the number of moles of the alumoxane that are employed is at least 100 times the number of moles of the metal-ligand complex general metal complex 1. When tris(pentafluorophenyl)borane alone is used as the activating co-catalyst, in some other embodiments, the number of moles of the tris(pentafluorophenyl)borane that are employed to the total number of moles of one or more metal-ligand complexes of general metal complex 1 form 0.5:1 to 10:1, in some other embodiments, from 1:1 to 6:1, in some other embodiments, from 1:1 to 5:1. The remaining activating co-catalysts are generally employed in approximately mole quantities equal to the total mole quantities of one or more metal-ligand complexes of general metal complex 1.

One or more features of the present disclosure are illustrated in view of the examples as follows:

### Example 1

### 1,3-dibromo-2-(2-ethoxyethoxy)-5-methylbenzene

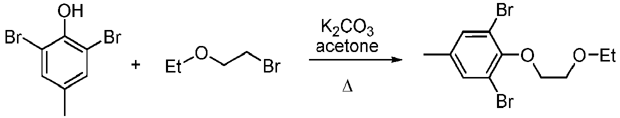

2,6-Dibromo-4-methylphenol (5.0 grams (g), 0.019 millimoles (mmol)) and 2-bromoethyl ethyl ether (2.9 g, 0.19 moles (mol)) were dissolved in 100 milliliters (mL) acetone. Potassium carbonate (K₂CO₃) was added to the solution, which turned purple within a minute. The mixture was stirred at reflux overnight. The mixture was cooled to room temperature, filtered, and reduced to an orange oil on the rotary evaporator. ¹H NMR spectrometric analysis indicated that there was still some unreacted starting phenol and ether present. The oil was taken up in 100 milliliter (mL) diethyl ether (Et₂O), and washed with 50 ml 1 molar (M) sodium hydroxide (NaOH) and 50 mL deionized water (H₂O). The ether portion was then dried with MgSO₄, filtered and reduced to a light orange oil (5.3 g, 83% yield). Both impurities were removed by the basic water workup.
¹H NMR (CDCl₃): δ = 7.28 (s, 2 H, Ar), 4.13 (t, J_{H-H} = 5.0 Hz, 2 H, OCH₂), 3.84 (t, J_{H-H} = 5.0 Hz, 2 H, OCH₂), 3.62 (q, J_{H-H} = 7.0 Hz, O*CH*₂CH₃), 2.25 (s, 3 H, aryl Me), 1.23 (t, J_{H-H} = 7.0 Hz, OCH₂C*H*₃). ¹³C NMR (CDCl₃): δ = 150.9, 136.5, 133.1, 117.7, 72.30, 69.4, 66.7, 20.2, 15.2.

### Example 2

### 1,3-dibromo-5-methyl-2-(3-phenoxypropoxy)benzene

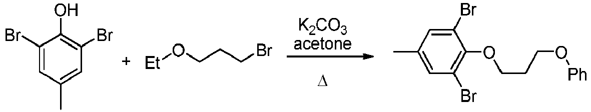

The phenol (5.0 g, 0.019 mol) and 1-Br-3-phenoxypropane (4.0 g, 0.019 mol) were dissolved in 100 mL acetone. K₂CO₃ was added to the solution, which turned purple within a minute. The mixture was stirred at reflux overnight. The mixture was cooled to room temperature, filtered, and reduced to an orange oil on the rotary evaporator. ¹H NMR spectrometric analysis indicated that there was still approximately 5% starting phenoxypropane. The oil was taken up in 100 mL Et₂O, and washed with 50 mL 1 M NaOH and 50 mL deionized H₂O. The ether portion was then dried with MgSO₄, filtered and reduced to a light orange oil (7.1 g, 94% yield).
¹H NMR (CDCl₃): δ = 7.28 (s, 2 H, aryl), 7.24-7.27 (m, 3 H, aryl), 6.92 (m, 3 H, aryl), 4.26 (t, J_{H-H} = 6.2 Hz, 2 H, OCH₂), 4.15 (t, J_{H-H} = 5.9 Hz, OCH₂), 2.30 (quint, J_{H-H} = 6.0 Hz, 2 H, CH₂C*H*₂CH₂), 2.25 (s, 3 H, aryl Me). ¹³C NMR (CDCl₃): δ =159.2, 151.0, 136.8, 133.3, 129.7, 129.6, 120.8, 118.0, 114.8, 70.0, 64.5, 30.2, 20.5.

### Example 3

### 2',4',6'-triisopropyl-2-(methoxymethoxy)-5-methylbiphenyl-3-ylboronic acid

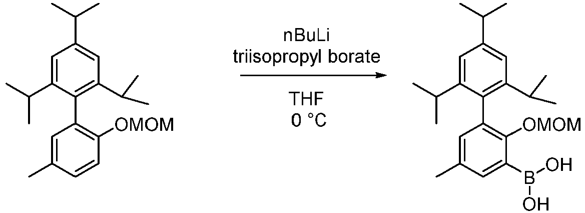

At 0 °C (degrees Celsius) and under nitrogen gas, 2,4,6-triisopropyl-2'-(methoxymethoxy)-5'-methylbiphenyl (500 mg, 1.41 mmol) in tetrahydrofuran (THF) (5 mL) was lithiated by adding butyllithium (n-BuLi) dropwise (2.5 molar (M) in hexanes, 728 microliter (µL), 1.82 mmol, 1.3 equivalent (eq)) to the stirred solution over a period of 10-15 minutes (min). The reaction held at this temperature for 3 hours (h). Triisopropyl borate (420 µL, 1.82 mmol, 1.3 eq) was added and the reaction stirred for an additional 1 h. The solution was warmed to room temperature and stirred overnight. The material was concentrated under reduced pressure and dissolved in 7 mL ice cold water. The solution was acidified with 2 normal (N) hydrochloric acid (HCl) extracted with methylene chloride. The extract was briefly dried over anhydrous sodium sulfate (Na₂SO₄) and filtered. The solvent was removed on the rotary evaporator. The crude product was obtained as a yellow oil and a quantitative yield, which was used directly in the following reactions.

### Example 4

### Pendant arm Ligand 11

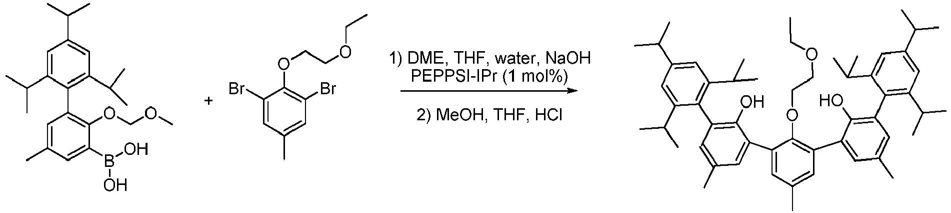

In the first step, the crude 2',4',6'-triisopropyl-2-(methoxymethoxy)-5-methylbiphenyl-3-ylboronic acid (approximately 0.70 mmol) was taken up in 5 mL dimethoxyethane (DME), which was distributed over two 10 mL reaction vials equipped with stirbar. One vial was used for this reaction. Cesium carbonate (Cs₂CO₃) (912 mg, 2.8 mmol, 4 eq), 1,3-dibromo-2-(2-ethoxyethoxy)-5-methylbenzene (108 milligrams (mg), 0.320 mmol, 0.45 eq), and water (1000 mL) were added, and nitrogen was bubbled through the mixture for 5 min. Finally, [1,3-Bis(2,6-Diisopropylphenyl)imidazol-2-ylidene](3-chloropyridyl)palladium(II) dichloride (PEPPSI™-iPr) (approximately 5 mg, approximately 7 µmol, 0.01 equivalents (eq)) was added to the mixture. The system was equipped with septum and purged with nitrogen. The tube was stirred at 60-65 °C overnight. The reaction was then concentrated under reduced pressure and extracted with 2 x 5 mL dichloromethane. The organic phase was dried over Na₂SO₄ and filtered. The solvent was removed on the rotary evaporator. The residue was taken up in 1 mL hexane and the methoxymethyl (MOM) protected reaction product purified by flash chromatography. A colorless oil was received in a yield of 100 mg (0.113 mmol), a 35% yield of 1,3-dibromo-2-(2-ethoxyethoxy)-5-methylbenzene.

In the second step, the oil was taken up in 1 mL THF, 5 mL MeOH and 2-3 drops of concentrated acetic acid added. The reaction was heated to reflux for 6 h, and left over night at 50 °C. Several drops of water were added, upon which the reaction mixture became hazy. The reaction was cooled to room temperature, upon which a precipitate formed, and left over night in the freezer. The supernatant was decanted, the residue washed with cold methanol and dried in the high vacuum. The pure product was received in form of a white powder in a yield of 54 mg (0.067 mmol), 60% yield with respect to MOM-protected starting material, and a 21% yield with respect to 1,3-dibromo-2-(2-ethoxyethoxy)-5-methylbenzene.
¹H NMR (500 MHz, CDCl₃): δ = 0.96 (t, ³*J* = 6.99 Hz, 3H, CH₂*CH*₃)*,* 1.086 (d, ³*J* = 6.86 Hz, 6 H, 2 x CH*CH*₃)*,* 1.091 (d, *J* = 6.86 Hz, 6 H, 2 x CH*CH*₃)*,* 1.30 (d, ³*J* = 6.93 Hz, 6 H, 2 x CH*CH*₃)*,* 2.34 (s, 6 H, 2 x Ar*CH*₃), 2.43 (s, 3 H, Ar*CH*₃), 2.68 (hept, ³*J* = 6.86 Hz, 4 H, 4 x C*HC*H₃), 2.92 (hept, ³*J* = 6.86 Hz, 2 H, 2 x HC*HC*H₃), 3.14 (t, ³*J* = 6.07 Hz, 2 H, *CH*₂-CH₂), 3.15 (q, ³*J* = 6.99 Hz, 2 H, *CH*₂CH₃), 3.63 (t, ³*J* = 6.07 Hz, 2 H, CH₂-*CH*₂), 5.65 (s, 2 H, 2 x OH), 6.88 (d, ⁴*J* = 2.09 Hz, 2 H, 2 x CH_{Ar}), 7.06 (s, 4 H, 4 x CH_{Ar}), 7.15 (d, ⁴*J =* 2.09 Hz, 2 H, 2 x CH_{Ar}), 7.23 (s, 2 H, 2 x CH_{Ar}) ppm. ¹³C NMR (126 MHz, CDCl₃): δ = 14.9, 20.6, 20.9, 24.0, 24.1, 24.5, 30.5, 34.3, 66.2, 68.5, 72.8, 121.0, 125.6, 127.7, 128.9, 130.5, 131.2, 131.6, 132.0, 132.1, 134.2, 147.4, 148.4, 148.8, 151.2.

### Example 4

### Pendant arm ligand 12

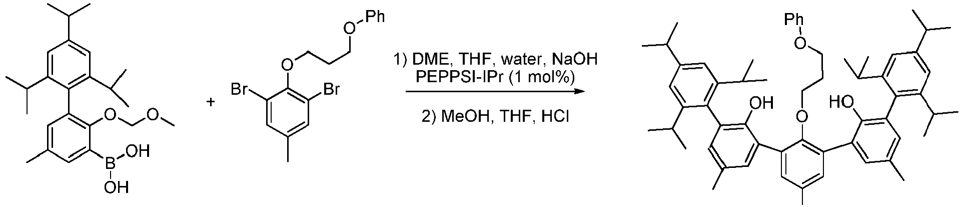

In the first reaction, the crude 2',4',6'-triisopropyl-2-(methoxymethoxy)-5-methylbiphenyl-3-ylboronic acid (approximately 0.70 mmol) was taken up in 5 mL DME, which was distributed over two 10 mL reaction vials equipped with stir bar. One vial was used for this reaction. Cs₂CO₃ (912 mg, 2.8 mmol, 4 eq), 1,3-dibromo-5-methyl-2-(3-phenoxypropoxy)benzene (128 mg, 0.320 mmol, 0.45 eq), and water (1000 mL) were added, and nitrogen was bubbled through the mixture for 5 min. Finally, PEPPSI-iPr (approximately 5 mg, approximately 7 µmol, 0.01 eq) was added to the mixture. The system was equipped with septum and purged with nitrogen. The tube was stirred at 60-65 °C over night. The reaction was then concentrated under reduced pressure and extracted with 2 x 5 mL dichloromethane. The organic phase was dried over Na₂SO₄ and filtered. The solvent was removed on the rotary evaporator. The residue was taken up in 1 mL hexane and the MOM-protected reaction product purified by flash chromatography. A colorless oil was received in a yield of 120 mg (0.127 mmol), a 40% yield with respect to 1,3-dibromo-5-methyl-2-(3-phenoxypropoxy)benzene.

In the second step, the oil was taken up in 1 mL THF, 5 mL MeOH and 2-3 drops of concentrated acetic acid added. The reaction was heated to reflux for 6 h, and left over night at 50 °C, after which a precipitate formed. The reaction was cooled to room temperature, and supernatant was decanted. The remaining residue washed with cold methanol and dried in the high vacuum. The pure product was received in form of a white powder in a yield of 62 mg (0.072 mmol), 23% yield of MOM-protected product, and a 21% yield of 1,3-dibromo-2-(2-ethoxyethoxy)-5-methylbenzene.

¹H NMR (500 MHz, CDCl₃): δ = 1.06 (d, ³*J* = 6.84 Hz, 6 H, 2 x CH*CH*₃)*,* 1.09 (d, ³*J* = 6.84 Hz, 6 H, 2 x CH*CH*₃)*,* 1.29 (d, ³*J* = 6.90 Hz, 6 H, 2 x CH*CH*₃)*,* 1.73 (p, ³*J* = 6.24 Hz, 2 H, CH₂C*H*₂CH₂), 2.31 (s, 6 H, 2 x Ar-*CH*₃), 2.43 (s, 3 H, Ar-*CH*₃), 2.67 (p, ³*J* = 6.84 Hz, 4 H, 4 x *CH*CH₃), 2.92 (d, ³*J* = 6.90 Hz, 2 H, 4 x *CH*CH₃), 3.48 (t, ³*J* = 6.45 Hz, 2 H, *CH*₂CH₂CH₂), 3.70 (t, ³*J* = 6.03 Hz, 2 H, CH₂CH₂*CH₂*), 5.68 (s, 2 H, OH), 6.64 (dd, ³*J =* 8.70, ⁴*J* = 0.96 Hz, 2 H, *ortho-Ph*)*,* 6.88 (td, ³*J* = 7.36 Hz, ⁴*J*=0.96 Hz, 1 H, *para-Ph*)*,* 6.90 (d, ⁴*J* = 2.13 Hz, 2 H, 2 x CH_{Ar}), 7.06 (s, 4 H, 4 x CH_{Ar}), 7.16 (d, ⁴*J* = 2.13 Hz, 2 H, 2 x CH_{Ar}), 7.20 (dd, J = 8.70, 7.36 Hz, 2 H, *meta-Ph*)*,* (s, 2 H, 2 x CH_{Ar}) ppm. ¹³C NMR (126 MHz, CDCl₃): δ = 20.6, 24.97, 24.03, 24.5, 29.5, 30.6, 34.3, 64.4, 71.3, 114.3, 120.4, 121.0, 125.7, 127.8, 129.1, 129.2, 130.4, 131.3, 131.5, 132.0, 132.1, 134.2, 147.4, 148.5, 148.8, 151.0, 158.7.

### Example 5

### 3-(9H-carbazol-9-yl)-2-(methoxymethoxy)-5-methylphenylboronic acid

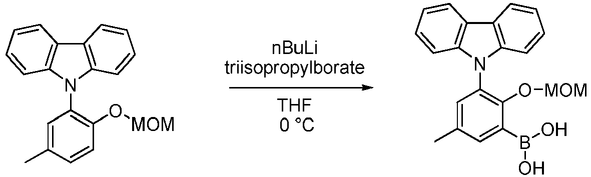

At 0° C and under nitrogen, 9-(2-(methoxymetyhoxy)-5-methylphenyl)-9*H-*carbazole (2.00 g, 6.30 mmol) in THF (18 mL) was lithiiated by adding nBuLi dropwise (2.5 M in hexanes, 3.27 mL, 8.19 mmol, 1.3 eq) to the stirred solution over a period of 10-15 min. The solution held at this temperature for 3 h. Triisopropyl borate (1.89 mL, 8.19 mmol, 1.3 eq) was added and stirred in ice for 1 h. The reaction was warmed to room temperature and stirred overnight. The mixture was concentrated on the rotrary evaporator and 50 mL ice water was added. The solution was acidified with 2 N HCl, while stirring and extracted with methylene chloride (3 x 50 mL). The extract was briefly dried over anhydrous Na₂SO₄ and filtered. The solvent was removed on the rotary evaporator. The crude product was obtained as a yellow oil and a quantitative yield, which was used directly in the following reactions.

### Example 6

### Pendant arm ligand 5

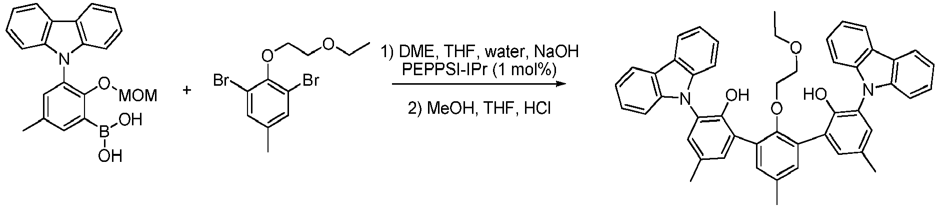

The crude 3-(9H-carbazol-9-yl)-2-(methoxymethoxy)-5-methylphenylboronic acid (approximately 3.15 mmol) was dissolved in 20 mL DME, which was distributed over two 50 mL roundbottom flasks with reflux condenser. One flask was used for this reaction. Cs₂CO₃ (4.11 g, 12.6 mmol, 4.0 eq), 1,3-dibromo-2-(2-ethoxyethoxy)-5-methylbenzene (426 mg, 1.26 mmol, 0.4 eq), and water (4 mL) were added, and the mixture was degassed (three times frozen in liquid nitrogen under nitrogen atmosphere, and thawed under vacuum). PEPPSI-iPr (45 mg, 0.063 mmol, 0.02 eq) was added to the vessel (N₂ counterflow). The system was heated to 60-65 °C over night, after which some black precipitate became visible. The mixture was cooled and extracted with dichloromethane (2 x 20 mL). The pooled organic phases were dried over Na₂SO₄ and filtered. The solvent was removed under reduced pressure. The residue was purified by flash chromatography. The main fraction contained the desired MOM-protected product (Mw=810.97 mg). The yield was 715 mg (0.882 mmol), a 70% yield of 2-(2-ethoxyethoxy)-5-methylbenzene), which was a white solid.

In a 100 mL roundbottom flask, 615 mg (0.759 mol) of the MOM-protected compound was dissolved in the least possible amount of THF. Next, 25 mL MeOH was added, upon which precipitation occurred. The stirred mixture was heated to 65 °C, and THF was then added dropwise until the residue dissolved. Next, 15 drops of concentrated HCl was added. The reaction was kept at 65 °C overnight. The flask was then cooled to -18 °C for two days after which crystallization occurred. The supernatant was pipetted off and the residue was washed with cold methanol (MeOH). The remaining solvent was removed under reduced pressure. The crystals were dried under high vacuum. This reaction yielded was 412 mg (0.570 mmol), a 75% yield, of the MOM protected product, which was a white powder. The structure of which was confirmed by NMR.
¹H NMR (500 MHz, CDCl₃) δ =0.84 (t, ³*J* = 7.00 Hz, 3 H, CH₂*Me*), 2.40 (s, 6 H, 2 x Ar-Me), 2.48 (s, 3 H, 1 x *Ar-Me*)*,* 3.13 (*q,* ³*J* = 7.00 Hz, 2 H, *CH*₂Me), 3.31 (t, ³*J* = 5.33 Hz, 2 H, CH₂*CH*₂), 3.77 (t, ³*J* = 5.33 Hz, 2 H, *CH*₂CH₂), 6.12 (s, 2 H, 2 x OH), 7.21-7.35 (m, 14 H, 6 x CH_{Ph}, 8 x CH_{Cbz}), 7.39 (ddd, ³*J* = 8.24, ³*J* =7.21, ⁴*J* = 1.18 Hz, 4 H, 4 x CH_{Cbz}), 8.14 (d, ³*J* = 7.65 Hz, 4 H, 2 x C4H_{Cbz}, 2 x C5H_{Cbz}) ppm. ¹³C NMR (126 MHz, CDCl₃): δ = 14.7, 20.53, 20.9, 66.41, 68.77, 73.43, 110.13, 119.84, 120.28, 123.47, 125.13, 125.87, 127.68, 129.43, 130.57, 131.32, 131.93, 132.51, 134.94, 141.22, 148.07, 150.82 ppm. HRMS (ESI⁺, m/z): calculated for [C₄₉H₄₂N₂O₄H]⁺: 723.3223, found 723.3188 (-3.4 mDa, 4.7ppm). MS/MS (ESI⁺, daughters of m/z=723): m/z= 317, 510, 677, 496, 302,180,167,194, 343, 485.

### Example 7

### Pendant arm ligand 10

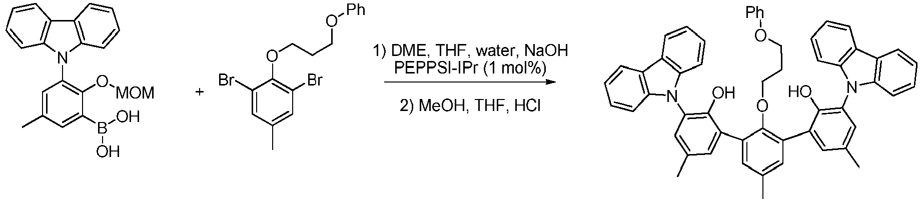

The crude 3-(9H-carbazol-9-yl)-2-(methoxymethoxy)-5-methylphenylboronic acid (approximately 3.15 mmol) was taken up in 20 mL DME, which was distributed over two 50 mL roundbottom flasks with reflux condenser. One flask was used for this reaction. CsCO₃ (4.11 g, 12.6 mmol, 4.0 eq), 1,3-dibromo-5-methyl-2-(3-phenoxypropoxy)benzene (504 mg, 1.26 mmol, 0.4 eq), and water (4 mL) were added, and the mixture was degassed (three times frozen in liquid N₂ under nitrogen atmosphere, and thawed under vacuum). PEPPSI-iPr (approximately 45 mg, 0.063 mmol, 0.02 eq) was added to the vessel (N₂ counterflow). The system was heated to 60-65 °C overnight, after which some black precipitate became visible. The mixture was cooled and extracted with dichloromethane (2 x 20 mL). The pooled organic phases were dried over Na₂SO₄ and filtered. The solvent was removed under reduced pressure. Three fractions were isolated by flash chromatography. The main fraction contained the desired MOM-protected product (M_{w}=873.04). The yield was 751 mg (0.860 mmol), with an overall yield of 68% yield of 3-phenoxzpopoxybenzene, which was a white, foamy solid.

In a 100 mL roundbottom flask, the 651 mg of the MOM-protected compound (0.745 mmol) was dissolved in the least possible amount of THF. 25 mL MeOH was added, upon which precipitation occurred. The stirred mixture was heated to 65 °C, and THF was added dropwise until the residue dissolved. 15 drops of concentrated HCl was added. The reaction was kept at 65 °C o-n. A yellow discoloration occurred and the flask was placed in the freezer. No crystallization occurred, so the mixture was dripped into water and the residue extracted into dichloromethane. The solvent was removed under reduced pressure and the residue recrystallized from methanol/tetrahydrofuran (THF). The crystals were dried under high vacuum. The yield was 414 mg (0.527 mmol), with an overall yield of 71% yield of a white powder. The structure of which was confirmed by NMR.
¹H NMR (500 MHz, CDCl₃) δ = 1.83 (p, ³*J* = 6.23 Hz, 2 H, CH₂C*H*₂CH₂), 2.35 (s, 6 H, 2 x Ar*Me*), 2.48 (s, 3 H, 1 x Ar*Me*), 3.65 (t, ³*J* = 6.23 Hz, , 2 H, *CH*₂CH₂CH₂), 3.80 (t, *J* = 6.23 Hz, 2 H, CH₂CH₂*CH*₂), 6.10 (s, 2 H, OH), 6.59 (dd, ³*J* = 8.02, ⁴*J* = 0.61 Hz, 2 H, *ortho-Ph*)*,* 6.85 (t, *J* = 7.31 Hz, 1 H, *para-Ph*), 7.12 (dd, *J* = 8.02, 7.31 Hz, 2 H, *meta-Ph*)*,* 7.16-7.36 (m, 18 H, 6 x CH_{Ph}, 12 x CH_{Cbz}), 8.12 (d, ³*J* = 7.72 Hz, 4 H, 2 x C4H_{Cbz}, 2 x C5H_{Cbz}). ¹³C NMR (126 MHz, CDCl₃): δ = 20.51, 20.93, 29.74, 64.04, 71.85, 110, 114.42, 119.91, 120.32, 120.66, 123.49, 124.98, 125.96, 127.4, 129.26, 129.52, 130.64, 131.33, 131.91, 132.52, 141.17, 148.01, 150.74, 158.46 ppm. HRMS (ESI⁺, m/z): calculated for [C₅₄H₄₅N₂O₄]⁺: 785.3379, found 785.3347(-3.2 mDa, 4.1ppm). MS/MS (ESI⁺, daughters of m/z=785): m/z= 317, 484, 135, 496, 302, 107, 167, 342, 651, 663.

### Example 8

### Hafnium Complex L10 (L10-Hf)

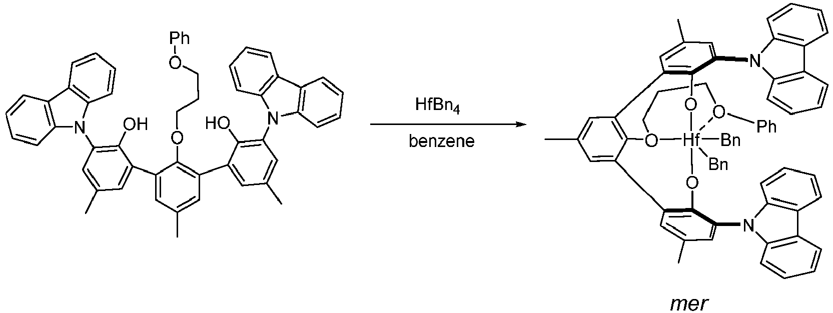

In a drybox, HfBn₄ (69.2 mg, 0.127 mmol, 1.00 eq) was dissolved in 600µL deuterobenzene. Pendant arm ligand **10** (100 mg, 0.127 mmol) was dissolved in 1.2 mL deuterobenzene. The HfBn₄ solution was added dropwise to the ligand solution, while stirring. The reaction was stirred in a closed vessel for 2 h and an NMR was taken, which indicated clean and quantitative conversion. The solvent was removed under high vacuum and the complex used as received. Detailed structural analysis was performed by NMR, confirming that the expected Cₛ-symmetric *mer* isomer was formed (FIG. 2).
¹H NMR (500 MHz, C₆D₆) δ = 0.70 (p, ³*J* = 6.03 Hz, 2 H, CH₂C*H*₂CH₂), 1.07 (s, 2 H, C*H*₂-Ph1), 1.72 (s, 2 H, C*H*₂-Ph3), 2.13 (s, 6 H, 2 x *ArMe*), 2.13 (s, 3 H, Ar*Me*), 2.88 (t, ³*J* = 6.03 Hz, 2 H, CH₂CH₂C*H*₂OPh-2), 3.26 (t, *J* = 6.03 Hz, 2 H, C*H*₂CH₂CH₂O-Ph2), 4.91 (dd, ³*J* = 7.74, ⁴*J* = 1.04 Hz, 2 H, *ortho-Ph-1*), 5.88 (t, ³*J* = 7.74 Hz, 2 H, *meta-Ph-1*), 6.28 (tt, ³*J* = 7.74, ⁴*J* = 1.04 Hz, 1 H, *para-Ph-1*), 6.55 (dd, ³*J* = 8.74, ⁴*J* = 1.04 Hz, 2 H, *ortho-Ph-2),* 6.73 (dd, ³*J* = 7.73, ⁴*J* = 1.16 Hz, 2 H, *ortho-Ph-3*)*,* 6.76 (tt, ³*J* = 7.73 Hz, ⁴*J* = 1.16 Hz, 1 H, *para-Ph-3*), 6.83 (tt, ³*J* = 7.45, ⁴*J* = 1.04 Hz, 1 H, *para-Ph-2*), 6.96 (t, ³*J* = 7.73 Hz, 2 H, *meta-Ph-3*), 7.07 (overlaid s, 6 H, 6 x CH_{Ar}), 7.11 (dd, ³*J* = 8.74, 7.45 Hz, 2 H, *meta-Ph-2),* 7.18 (m, 2 H, 2 x C6-cbz-*H*), 7.21 (ddd, ³*J* = 7.9, 7.0 Hz, ⁴*J* = 0.9 Hz, 2H, 2 x C3-cbz-*H*), 7.31-7.28 (m, 4 H, 2 x C7-cbz-*H*, 2 x C8-cbz-*H*), 7.39 (ddd, ³*J* = 8.25, 7.0 Hz, ⁴*J* = 1.18 Hz, 2 H, 2 x C2-cbz-*H*), 7.49 (dd, ³*J* = 8.25 Hz, ⁴*J* = 0.9, 2 H, 2 x C1-cbz-*H*), 8.00 (dd, ³*J* = 7.9 Hz, ⁴*J* =1.18 Hz, 4 H, 2 x C4-cbz-*H*, 2 *x* C5-cbz-*H*) ppm; ¹³C NMR (126 MHz, CDCl₃): δ = 20.53, 21.36, 28.71, 63.08, 70.95, 71.32, 75.05, 109.66, 112.7, 114.69, 120.04, 120.12, 120.46, 121.05, 121.16, 121.25, 123.39, 124.35, 125.74, 126.41, 126.74, 127.31, 128.02, 128.29, 128.99, 129.28, 129.63, 130, 130.16, 130.56, 133.27, 135.37, 138.56, 139.71, 141.31, 141.89, 154.67, 158.87 ppm.

### Example 9

### Hafnium Complex L5 (L5-Hf)

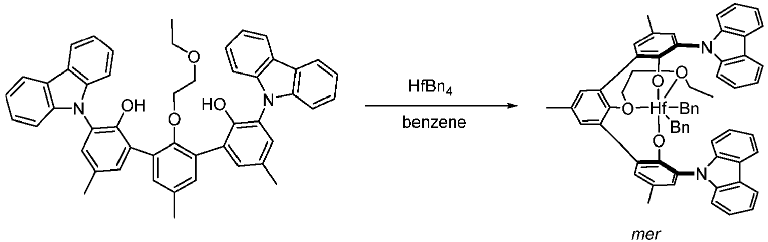

In a drybox, tetrabenzyl hafnium (HfBn₄) (37.6 mg, 69.2 µmol, 1.00 eq) was dissolved in 600 µL deuterobenzene. Pendant arm ligand **5** (50.0 mg, 69.2 µmol) was dissolved in 1.2 mL deuterobenzene. The HfBn₄ solution was added dropwise to the ligand solution, while stirring. The reaction was stirred in a closed vessel for 2 h, upon which the product precipitated. The resulting complex is completely insoluble in deuterobenzene. A 100 mL aliquot was therefore diluted with CD₂Cl₂ and NMR taken. NMR confirmed expected, Cₛ-symmetric product. The supernatant was pipetted off and the remaining residue washed with toluene. The remaining solvent was removed under high vacuum, providing a quantitative yield. Detailed structural analysis was performed by NMR, confirming that the expected Cₛ-symmetric *mer* isomer was formed.
¹H NMR (500 MHz, CD₂Cl₂) δ = -0.27 (t, ³*J* = 6.94 Hz, 3 H, OCH₂*Me*), 0.42 (s, 2 H, C*H*₂Ph-1, 1.03 (s, 2 H, C*H*₂Ph-2), 2.18 (q, ³*J* = 6.94 Hz, 2 H, OC*H*₂Me), 2.45 (s, 6 H, 2 x Me), 2.48 (s, 3 H, 1 x Me), 2.96 (t, ³*J* = 5.10 Hz, 2 H, C*H*₂CH₂), 3.58 (t, ³*J* = 5.10 Hz, 2 H, CH₂*CH*₂), 4.85 (dd, ³*J* = 7.2, ⁴*J* = 2.3 Hz, 2 H, *ortho-Ph-1*), 6.13 (dd, ³*J* = 8.1, ⁴*J* = 1.2 Hz, 2 H, *ortho-Ph-2*)*,* 6.27 (m, 3 H, *meta, para-Ph-1*), 6.60 (tt, *J* = 7.4, 1.2 Hz, 1 H, *para-Ph-2*), 6.82 (dd, *J* = 8.1, 7.4 Hz, 2 H, *meta-Ph-2*), 7.15-7.40 (m, 18 H, 6 x CH_{Ar}, 12 x CH_{cbz}) 8.12 (ddd, ³*J* = 7.78, ⁴*J* = 1.21, ⁵*J* = 0.77, 2 H, C₄H_{cbz}), 8.14 (ddd, ³*J* = 7.81, ⁴*J* = 1.25, ⁵*J* = 0.75 Hz, 2H, C5H_{cbz}) ppm; ¹³C NMR (126 MHz, CDCl₃): δ = 12.55, 20.9, 21.99, 66.83, 67.23, 71.34, 72.59, 74, 109.72, 112.72, 119.85, 119.99, 120.15, 120.39, 120.8, 120.97, 123.28, 124.17, 125.85, 126.53, 126.71, 126.96, 127.53, 127.66, 127.71, 129.5, 129.68, 130.6, 131.8, 133.31, 134.87, 140.43, 141.81, 141.88, 145.76, 149.27, 154.87 ppm.

### Example 10

### Zr complex L10 (L10-Zr)

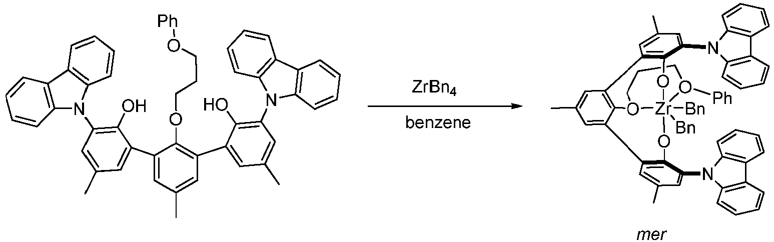

In a drybox, ZrBn₄ (58.1 mg, 0.127 mmol, 1.00 eq) was dissolved in 600 µL deuterobenzene. Pendant arm ligand **10** (100 mg, 0.127 mmol) was dissolved in 1.2 mL deuterobenzene. The ZrBn₄ solution was added dropwise to the ligand solution, while stirring. The reaction was stirred in a closed vessel overnight and an NMR was taken *in situ,* confirming formation of the product. The zirconium complex was precipitated from hexanes, the solvent decanted and the pure complex dried under high vacuum. Detailed structural analysis was performed by NMR, confirming that the expected Cₛ-symmetric *mer* isomer was formed.
¹H NMR (500 MHz, C₆D₆) δ = 0.79 (s, 2 H, C*H*₂-Ph1), 1.16 (p, ³*J* = 5.99 Hz, H, CH₂C*H*₂CH₂), 1.31 (s, 2 H C*H*₂-Ph3), 2.12 (s, 6 H, 2 x Ar*Me*), 2.20 (s, 1 H, Ar*Me*)*,* 3.10 (t, ³*J* = 6.08 Hz, 2 H, CH₂CH₂C*H*₂OPh-2), 3.57 (t, ³*J* = 6.08 Hz, 2 H, C*H*₂CH₂CH₂OPh-2), 4.78 (dd, ³*J* = 7.8 Hz, ⁴*J* = 0.8 Hz, 2 H, *ortho-Ph1*), 6.19 (t, *J* = 7.8 Hz, 2 H, *meta-Ph1*), 6.47 (dd, ³*J* = 7.8 Hz, ⁴*J* = 1.26 Hz, 2 H, *ortho-Ph3*), 6.47 (tt, *J* = 7.8 Hz, ⁴*J* = 0.8 Hz, 1 H, *para-Ph1*), 6.63 (dd, ³*J* = 8.0, ⁴*J* = 1.06 Hz, 2 H, *ortho-Ph2*)*,* 6.62-6.66 (m, overlaid, 1 H, *para-Ph3*), 6.69 (t, ³*J* = 7.8 Hz, 2 H, *meta-Ph3*), 6.84 (tt, ³*J* = 8.0 Hz, 1.06 Hz, 1 H, *para-Ph2*), 6.93-6.94 (overlaid s, 2 H, 2 x CH_{Ar}), 7.09 (overlaid s, 2 H, 2 x CH_{Ar} (middle ring)), 7.12 (t, ³*J* =8.0 Hz, 1 H, *meta-Ph2*), 7.14 (overlaid s, 2 H, 2 x CH_{Ar}), 7.12-7.15 (t, hidden, visible by 1D-TOCSY, 2 H, 2 x C3-H_{Cbz}) 7.20 (m, 2 H, 2 x C6-H_{Cbz}), 7.22 (dt, ³*J* = 8.05, ⁴*J* = 0.75, 2 H, 2 x C1-H_{Cbz}), 7.27-7.32 (m, 6 H, 2 x C2-H_{Cbz}, 2 x C7-H_{Cbz}, 2 x C8-H_{Cbz}), 7.99 (dd, ³*J* = 7.77 Hz, ⁴*J*=0.8 Hz, 2 H, 2 x C4-H_{Cbz}), 8.03 (dd, ³*J* = 7.72 Hz, ⁴*J*=0.83 Hz, 2 H, 2 x C5-H_{Cbz}) ppm; ¹³C NMR (126 MHz, CDCl₃): δ = 20.5, 21.26, 29.16, 59.46, 59.64, 63.34, 75.45, 109.67, 111.64, 114.77, 119.88, 119.97, 120.54, 120.97, 121.15, 121.67, 123.43, 123.9, 125.79, 125.9, 126.31, 127.93, 128.3, 128.92, 129.25, 129.4, 129.66, 130.26, 130.8, 130.92, 131.01, 132.99, 135.2, 138.07, 139.16, 139.59, 141.78, 142.09, 155.22, 158.96 ppm.

### Example 11

### Zr Complex L5 (L5-Zr)

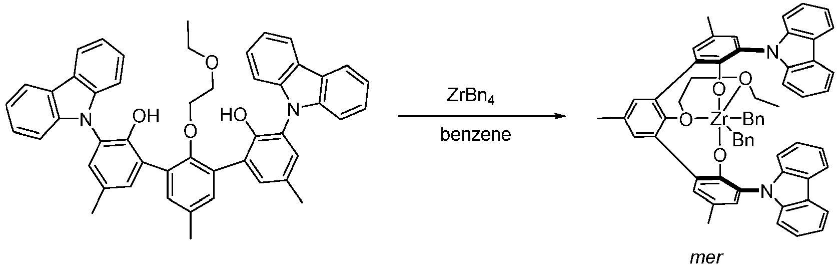

In a drybox, ZrBn₄ (63.0 mg, 0.138 mmol, 1.00 eq) was dissolved in 600 µL deuterobenzene. Pendant arm ligand **5** (100 mg, 0.138 mmol) was dissolved in 1.2 mL deuterobenzene. The ZrBn₄ solution was added dropwise to the ligand solution, while stirring. The reaction was stirred in a closed vessel overnight and an NMR was taken *in situ,* confirming formation of the product. The zirconium complex was precipitated from hexanes, the solvent decanted and the pure complex dried under high vacuum. Detailed structural analysis was performed by NMR, confirming that the expected symmetric *mer* isomer was formed.
¹H NMR (500 MHz, C₆D₆) δ = 0.56 (t, ³*J* = 6.97 Hz, OCH₂*Me*), 0.76 (s, 2 H, C*H*₂-Ph-1), 1.58 (s, 2 H, C*H*₂-Ph-2), 2.15 (s, 6 H, 2 x Me), 2.20 (s, 3 H, 2 x Me), 2.58-2.53 (m (possibly overlaid dd, ³*J* = 3.26, 5.98 (t, ³*J* = 4.6), 2 H, CH₂C*H*₂OCH₂), 2.62 (q, ³*J* = 6.97 Hz, 2 H, OC*H*₂Me), 3.30-3.25 (m, (possibly overlaid dd, ³*J* = 3.26,5.98 and t, ³*J* = 4.6), 2 H, C*H*₂CH₂OCH₂), 5.02 (dd, ³*J* = 7.9, ⁴*J* = 1.1 Hz, 2 H, *ortho-*Ph-1)*,* 6.36 (t, *J* = 7.9 Hz, 2 H, *meta*-Ph-1), 6.45 (d, ³*J* = 7.3 Hz, 2 H, *ortho*-Ph-2)*,* 6.47 (tt, ³*J* = 7.9 Hz, ⁴*J* = 1.1 Hz, 1 H, *para*-Ph-1), 6.65 (tt, ³*J* = 7.3 Hz, ⁴*J* = 1.6 Hz, 1 H, *para*-Ph-2), 6.71 (t, ³*J* = 7.3 Hz, *meta-*Ph-2), 6.96 (d, ⁴*J* = 0.56 Hz, 2 H, 2 x C*H*_{Ar}), 7.13 (d, ⁴*J* = 0.56 Hz, 2 H, 2 x C*H*_{Ar} (center ring)), 7.14-7.18 (m, 4 H, 2 x C3H_{cbz}, 2 x C*H*_{Ar}), 7.22 (ddd, ³*J* = 7.8, *J* = 4.64, ⁴*J*=3.47 Hz, 2 H, 2 x C6*H*_{cbz}), 7.26-7.35 (m, 8 H, 2 x C2*H*_{cbz}, 2 x C1*H*_{cbz}, 2 x C7*H*_{cbz}, 2 x C8*H*_{cbz}), 8.00 (d, ³*J* = 7.8 Hz, 2 H, 2 x C4*H*_{cbz}), 8.05 (d, ³*J* = 7.8, 2 H, 2 x C5*H*_{cbz}) ppm; ¹³C NMR (126 MHz, CDCl₃): δ = 14.43, 20.46, 21.28, 60.92, 61.45, 66.39, 68.69, 76.25, 109.65, 111.9, 119.86, 119.92, 120.54, 120.99, 121.86, 122.26, 123.38, 124.06, 125.61, 125.89, 126.44, 127.93, 128.31, 128.51, 128.81, 129.5, 129.83, 130.35, 130.65, 131.17, 132.79, 135.08, 139.06, 140.23, 141.29, 141.86, 142.09, 142.85, 155.19 ppm.

### High Temperature Gel Permeation Chromatography (HT GPC):

A high temperature Gel Permeation Chromatography system (GPC IR) consisting of an Infra-red concentration detector (IR-5) from PolymerChar Inc (Valencia, Spain) was used for Molecular Weight (MW) and Molecular Weight Distribution (MWD) determination. The carrier solvent was 1,2,4-trichlorobenzene (TCB). The auto-sampler compartment was operated at 160 °C, and the column compartment was operated at 150 °C. The columns used were four Polymer Laboratories Mixed A LS, 20 micron columns. The chromatographic solvent (TCB) and the sample preparation solvent were from the same solvent source with 250 ppm of butylated hydroxytoluene (BHT) and nitrogen sparged. The samples were prepared at a concentration of 2 mg/mL in TCB. Polyethylene samples were gently shaken at 160 °C for 2 hours. The injection volume was 200 µl, and the flow rate was 1.0 ml/minute.

Calibration of the GPC column set was performed with 21 narrow molecular weight distribution polystyrene standards. The molecular weights of the standards ranged from 580 to 8,400,000 g/mol, and were arranged in 6 "cocktail" mixtures, with at least a decade of separation between individual molecular weights.

The polystyrene standard peak molecular weights were converted to polyethylene molecular weights using the following equation (as described in Williams and Ward, J. Polym. Sci., Polym. Let., 6, 621 (1968)): ${M}_{polyethylene} = A {\left({M}_{polystyrene}\right)}^{B}$

Here B has a value of 1.0, and the experimentally determined value of A is around 0.42.

A third order polynomial was used to fit the respective polyethylene-equivalent calibration points obtained from equation (1) to their observed elution volumes of polystyrene standards.

Number, weight, and z-average molecular weights were calculated according to the following equations: $\overline{Mn} = \frac{{\sum }_{ }^{i} {Wf}_{i}}{{\sum }_{ }^{i} \left(\frac{{Wf}_{i}}{{M}_{i}}\right)}$ $\overline{Mw} = \frac{{\sum }_{ }^{i} \left({Wf}_{i}∗{M}_{i}\right)}{{\sum }_{ }^{i} {Wf}_{i}}$ $\overline{Mz} = \frac{{\sum }_{ }^{i} \left({wf}_{i}∗{{M}_{i}}^{2}\right)}{{\sum }_{ }^{i} \left({Wf}_{i}∗{M}_{i}\right)}$

Where, *Wfᵢ* is the weight fraction of the *i*-th component and *Mᵢ* is the molecular weight of the *i*-th component.

The MWD was expressed as the ratio of the weight average molecular weight (Mw) to the number average molecular weight (Mn).

The accurate A value was determined by adjusting A value in equation (1) until Mw calculated using equation (3) and the corresponding retention volume polynomial, agreed with the known Mw value of 120,000 g/mol of a standard linear polyethylene homopolymer reference.

### GPC Deconvolution:

The GPC data was deconvoluted to give the most probable fit for two molecular weight components. There are a number of deconvolution algorithms available both commercially and in the literature. These may lead to different answers depending upon the assumptions used. The algorithm summarized here is optimized for the deconvolution problem of the two most probable molecular weight distributions (plus an adjustable error term). In order to allow for the variations in the underlying distributions due to the macromer incorporation and small fluctuations in the reactor conditions (i.e. temperature, concentration) the basis functions were modified to incorporate a normal distribution term. This term allows the basis function for each component to be "smeared" to varying degrees along the molecular weight axis. The advantage is that in the limit (low LCB, perfect concentration and temperature control) the basis function will become a simple, most probable, Flory distribution.

Three components (j=1,2,3) are derived with the third component (j=3) being an adjustable error term. The GPC data must be normalized and properly transformed into weight fraction versus Log₁₀ molecular weight vectors. In other words, each potential curve for deconvolution should consist of a height vector, hᵢ, where the heights are reported at known intervals of Log₁₀ molecular weight, the hᵢ have been properly transformed from the elution volume domain to the Log₁₀ molecular weight domain, and the hᵢ are normalized. Additionally, these data should be made available for the Microsoft EXCEL™ application.

Several assumptions are made in the deconvolution. Each component, j, consists of a most probable, Flory, distribution which has been convoluted with a normal or Gaussian spreading function using a parameter, σⱼ. The resulting, three basic functions are used in a Chi-square, X², minimization routine to locate the parameters that best fit the n points in hᵢ , the GPC data vector. ${x}^{2} \left({μ}_{j}, {σ}_{j}, {w}_{j}\right) = {\sum }_{i = 1}^{n} {\left[{\sum }_{j = 1}^{3} ⋅ {\sum }_{k = 1}^{20} {w}_{j} ⋅ {M}_{i}^{2} ⋅ {λ}_{j , k}^{2} ⋅ {CumND}_{j , k} ⋅ {e}^{- {λ}_{j , k} ⋅ {M}_{i}} ⋅ Δ {Log}_{10} M - {h}_{i}\right]}^{2} {λ}_{j , k} = {μ}_{j} + \frac{k - 10}{3} ⋅ {σ}_{j}$

The variable, CumND_{j,k}, is calculated using the EXCEL™ function "NORMDIST( x, mean, standard_dev, cumulative)" with the parameters set as follows: $x = {μ}_{j} + \left(k-10\right) ∗ {σ}_{j} / 3$
mean = µⱼ
standard dev = σⱼ
cumulative = TRUE

Table 1 below summarizes these variables and their definitions. The use of the EXCEL™ software application, Solver, is adequate for this task. Constraints are added to Solver to insure proper minimization.

**Table 1: Variable Definitions**

| Variable Name | Definition |
|---|---|
| λ_{j,k} | Reciprocal of the number average molecular weight of most probable ( Flory ) distribution for component j, normal distribution slice k |
| σⱼ | Sigma (square root of variance) for normal (Gaussian) spreading function for component j. |
| wⱼ | Weight fraction of component j |
| K | Normalization term (1.0 / Logₑ 10) |
| Mᵢ | Molecular weight at elution volume slice i |
| hᵢ | Height of log₁₀ (molecular weight) plot at slice i |
| n | Number of slices in Log molecular weight plot |
| i | Log molecular weight slice index (1 to n) |
| j | Component index (1 to 3) |
| l. k | Normal distribution slice index |
| Δlog₁₀M | Average difference between log₁₀Mᵢ and log₁₀Mᵢ₋₁ in height vs. log₁₀M plot |

The 8 parameters that are derived from the Chi-square minimization are µ₁, µ₂, µ₃, σ₁, σ₂, σ₃, w₁, and w₂. The term w₃ is subsequently derived from w₁ and w₂ since the sum of the 3 components must equal 1. Table 2 is a summary of the Solver constraints used in the EXCEL program.

**Table 2: Constraint summary**

| **Description** | **Constraint** |
|---|---|
| Maximum of fraction 1 | w₁ < 0.95 (User adjustable) |
| Lower limit of spreading function | σ₁, σ₂, σ₃ > 0.001 (must be positive) |
| Upper limit of spreading function | σ₁, σ₂, σ₃ < 0.2 (User adjustable) |
| Normalized fractions | w₁ + w₂ + w₃ =1.0 |

Additional constraints that are to be understood include the limitation that only µⱼ > 0 are allowed, although if Solver is properly initialized, this constraint need not be entered, as the Solver routine will not move any of the µⱼ to values less than about 0.005. Also, the wⱼ are all understood to be positive. This constraint can be handled outside of Solver. If the wⱼ are understood to arise from the selection of two points along the interval 0.0 < P₁ < P₂ <1.0; whereby w₁ =P₁, w₂ =P₂- P₁ and w₃ = 1.0 - P₂; then constraining P₁ and P₂ are equivalent to the constraints required above for the wⱼ.

Table 3 is a summary of the Solver settings under the Options tab.

**Table 3: Solver settings**

| **Label** | **Value or selection** |
|---|---|
| Max Time (seconds) | 1000 |
| Iterations | 100 |
| Precision | 0.000001 |
| Tolerance (%) | 5 |
| Convergence | 0.001 |
| Estimates | Tangent |
| Derivatives | Forward |
| Search | Newton |
| ALL OTHER SELECTIONS | Not selected |

A first guess for the values of µ₁, µ₂, w₁, and w₂ can be obtained by assuming two ideal Flory components that give the observed weight average, number average, and z-average molecular weights for the observed GPC distribution. ${M}_{n , GPC} = {\left[{w}_{1}⋅\frac{1}{{10}^{{μ}_{1}}}+{w}_{2}⋅\frac{1}{{10}^{{μ}_{2}}}\right]}^{- 1}$ ${M}_{w , GPC} = \frac{\left[{w}_{1}⋅2⋅{10}^{{μ}_{1}}+{w}_{2}⋅2⋅{10}^{{μ}_{2}}\right]}{{M}_{n , GPC}}$ ${M}_{z , GPC} = \frac{\left[{w}_{1}⋅6⋅{10}^{{μ}_{1}}+{w}_{2}⋅6⋅{10}^{{μ}_{2}}\right]}{{M}_{w , GPC}}$ ${w}_{1} + {w}_{2} = 1$

The values of µ₁, µ₂, w₁, and w₂ are then calculated. These should be adjusted carefully to allow for a small error term, w₃, and to meet the constraints in Table 2 before entering into Solver for the minimization step. Starting values for σⱼ are all set to 0.05.

Some of the embodiments of general metal complex 1 are used as catalysts in ethylene-octene copolymerization reactions. Table 4 lists the results of uptakes and yields for four different catalysts: Comparative Catalyst 1 (CC 1), hafnium tetrabenzyl (Hf(Benz)₄, L10-Hf, and Comparative Catalyst 2 (CC 2). The L10-Hf has the general metal complex 1 structure. The other listed catalysts are known, and Hf(Benz)₄ is the metal precursor for L10-Hf catalyst.

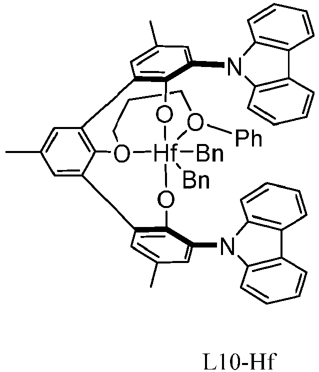

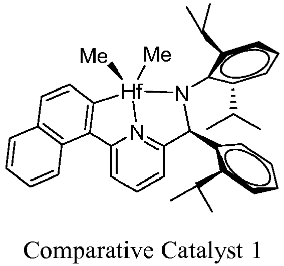

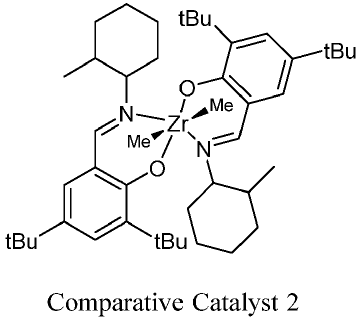

Catalyst L10-Hf was tested to determine its abilities in comparison with controls - known catalysts. The polymerization reaction was conducted at 120 °C, with an ethylene pressure of 1.4 MPa (200 psig), and 1-octene was loaded at 300 µL. The metal catalyst, activator, and scavenger were added in 1 to 2.4 to 20 ratio, wherein the activator is RIBS-2 (tetrakis(pentafluorophenyl)borate(1-) amine), and the scavenger is MMAO (modified methylalumoxane). Hf(Benz)₄ was the metal precursor and did not perform well, yielding overall poor results. CC 1 is a pyridyl amide catalyst and a standard comparative catalyst. In comparison, L10-Hf has a similar uptake and having comparative molecular weights (Mw) and molecular distribution (PDI).

**Table 4**

| **Catalyst** | **Uptake** | **Quench Time (s)** | **Yield (g)** | **Mw** | **Num MW** | **PDI** | **µmol (catalyst)** |
|---|---|---|---|---|---|---|---|
| CC 1 | 71.43 | 35.4 | 0.2365 | 7.19 E+05 | 1.25 E +05 | 5.78 | 2818 |
| CC 1 | 75.24 | 47.4 | 0.2191 | 8.24 E +05 | 1.20 E +05 | 6.89 | 2818 |
| Hf(Benz)₄ | 21.06 | 1200 | 0.0234 | | | | 4697 |
| Hf(Benz)₄ | 19.69 | 1202 | 0.022 | | | | 4697 |
| L10-Hf | 70.97 | 96.1 | 0.1741 | 9.02 E +04 | 1.86 E +04 | 4.86 | 4697 |
| L10-Hf | 70.51 | 134.5 | 0.1579 | 1.09 E +05 | 2.12 E +04 | 5.12 | 4697 |
| CC 2 | 73.41 | 67.7 | 0.1374 | 8.65 E +05 | 3.03 E +05 | 2.85 | 939 |
| CC 2 | 70.97 | 58.6 | 0.1242 | 1.03 E +06 | 3.11 E +05 | 3.30 | 939 |

While the catalyst does not have to activity of the CC 1, the present pyridyl amide is valuable in that synthesis is simpler at the industrial level. Thus, suitable industrial scale catalysts, like the present terphenyl catalysts, have significant value, especially for products like adhesives. Batch reactions of ethylene-octene copolymerization studies were performed with different catalysts; where imino-enanido Comparative Catalyst 3 (CC 3) was the known control, and L5-Hf and L5-Zr were the tested catalysts. The results are shown in Table 5 and Table 6. The batch reactions listed in Tables 5 and 6 were performed under the same conditions with varying catalysts and catalyst amounts. The polymerization reactions were stirred for 10 minutes with 250 gram of 1-octene, at 120 °C, with 1.2 eq of RIBS-2, and 10 eq of MMAO, the scavenger.

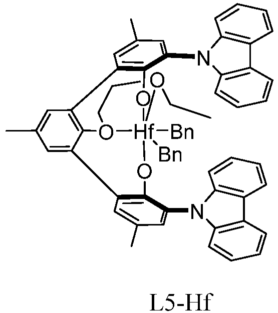

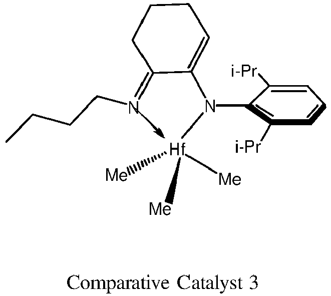

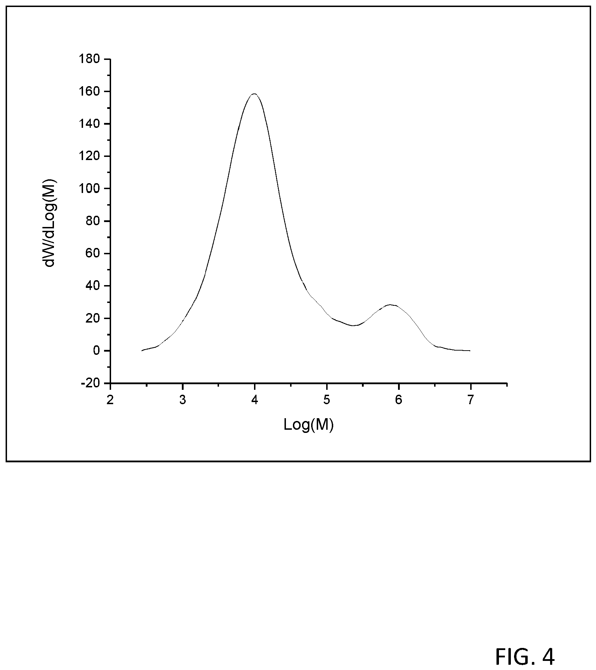

The results shown in FIG. 4 were evidence that L10-Hf has bimodal properties and the large molecular weight distribution, which is critical for good adhesive qualities. Both the zirconium and the hafnium L10 catalyst (L10-Hf and L10-Zr) were able to incorporate a higher mole percent of octene than the comparison, CC 3, though the molecular weight was lower. Overall, the L10 catalyst yielded polymers with good adhesive properties, with a large PDI, and good yields.

**Table 5**

| Catalyst | | | Exotherm (°C) | Ethylene (g) added | Yield (g) | Efficiency gpoly/gMetal | Tm (°C) | Mw | Mw/Mn | Density | Mol% Octene |
|---|---|---|---|---|---|---|---|---|---|---|---|
| Name | µmol | Metal | | | | | | | | | |
| CC 3 | 0.3 | Hf | 1.0 | 9.6 | 18.4 | 343,623 | 86.8 | 1,152,790 | 6.19 | 0.896 | 6.826 |
| CC 3 | 0.3 | Hf | 1.4 | 9.6 | 33.3 | 621,886 | 85.6 | 1,181,460 | 5.01 | 0.882 | 9.084 |
| L10-Hf | 2.0 | Hf | 1.6 | 18.1 | 31.2 | 34,960 | 76.3/105.6 | 126,617 | 20.66 | 0.852 | 15.593 |
| L10-Hf | 3.0 | Hf | 0.7 | 12.6 | 23.5 | 43,887 | 73.8/104.7 | 179,277 | 31.10 | 0.855 | 14.927 |
| L10-Zr | 10.0 | Zr | 3.1 | 30.6 | 57.7 | 210,836 | 16.9/106.7 | 4,519 | 2.18 | 0.845 | 17.587 |

As the results show in Table 6, L10-Zr and L5-Zr incorporate more 1-octene than the other known catalysts, even though a higher amount of catalyst was used to achieve the greater octene incorporation.

**Table 6**

| Catalyst | | | Exotherm (°C) | Ethylene (g) added | Yield (g) | Efficiency gpoly/gMetal | Tm (°C) | Mw | Mw/Mn | Density | Mol % Octene |
|---|---|---|---|---|---|---|---|---|---|---|---|
| Name | µmol | Metal | | | | | | | | | |
| CC 3 | 0.3 | Hf | 1.1 | 8.7 | 23.6 | 440,734 | 86.7 | 1,064,530 | 52.46 | 0.886 | 8.455 |
| CC 3 | 0.3 | Hf | 0.9 | 8.5 | 27.9 | 521,038 | 86.5 | 1,420,230 | 2.63 | 0.900 | 6.183 |
| L10-Zr | 2.0 | Zr | 1.8 | 19.9 | 36.8 | 201,701 | 15.9/51.6 | 4,595 | 2.19 | 0.845 | 17.620 |
| L5-Zr | 3.0 | Zr | 0.9 | 1.6 | 4.3 | 15,712 | -10.6 | 24,069 | 4.32 | 0.843 | 18.182 |
| L5-Zr | 10.0 | Zr | 0.6 | 4.4 | 9.5 | 10,414 | -10.4 | 27,548 | 2.12 | 0.834 | 21.265 |

Unless otherwise defined, all technical and scientific terms used in this disclosure have the same meaning as commonly understood by one of ordinary skill in the art. The terminology used in the description is for describing particular embodiments only and is not intended to be limiting. As used in the specification and appended claims, the singular forms "a," "an," and "the" are intended to include the plural forms as well, unless the context clearly indicates otherwise.

As used in this disclosure, "approximately" means near or approaching a certain state, such as degrees, reaction conditions or molecular weights. Additionally, the disclosure of any ranges in the specification and claims are to be understood as including the range itself and also anything subsumed within the range, as well as endpoints. Unless otherwise indicated, the numerical properties set forth in the specification and claims are approximations that may vary depending on the desired properties sought to be obtained in embodiments of the present disclosure. Notwithstanding that numerical ranges and parameters setting forth the broad scope of the disclosure are approximations, the numerical values set forth in the specific examples are reported as precisely as possible. Any numerical values, however, inherently contain certain errors necessarily resulting from error found in their respective measurements.

## Claims

1. A metal complex comprising the following structure: 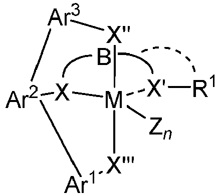 wherein
at least two of the following bonds X-M, X'-M, X"-M, and X"'-M are covalent and at least one is dative; the M-X' bond is covalent, dative bond or does not exist;
Ar¹, Ar², and Ar³ are independently an aryl moiety or heteroaryl moiety;
B is an aliphatic moiety or heteroaliphatic moiety linking X and X', wherein B has from one to 50 atoms, not counting H;
X is oxygen, sulfur, substituted nitrogen, substituted phosphorus, or substituted carbon;
X' is oxygen, sulfur, substituted nitrogen, substituted phosphorus, or an unsaturated carbon group;
X" and X'" are independently selected from oxygen, sulfur, substituted nitrogen, or substituted phosphorus,
R¹ is either: 1) an aliphatic moiety, a heteroaliphatic moiety, an aromatic moiety, or a heteroaromatic moiety; or 2) part of an aliphatic carbocycle, an aromatic carbocycle, a heterocyclic moiety, or a heteroaromatic moiety; and
M is one or more metals selected from a Group 4 element;
wherein the terms "substituted nitrogen" is a tertiary, secondary or primary nitrogen moiety and "substituted phosphorus" is a tertiary, secondary or primary phosphorous moiety; and
the term "substituted carbon" is wherein at least one hydrogen atom bound to said carbon atom is replaced with one or more substituents that are functional groups selected from hydroxyl, alkoxyl, alkylthio, amino, halo and silyl.

2. The metal complex of claim 1, wherein Ar¹ and Ar³ are substituted with 1-4 hydrogens, 1-4 aromatic moieties, 1-4 aliphatic moieties, 1-4 substituted heteroatoms, or 1-4 halide moieties, and Ar² is substituted with 1-3 hydrogens, 1-3 aromatic moieties, 1-3 aliphatic moieties, 1-3 substituted heteroatoms, or 1-3 halide moieties.

3. The metal complex of claims 1 or 2, wherein Zₙ includes 1-4 independent moieties that are either identical or different from one another, and wherein the Zₙ moieties form covalent bonds, dative bonds, ionic bonds, or combinations thereof with M.

4. The metal complex of any preceding claim, wherein the metal complex has the following structure: 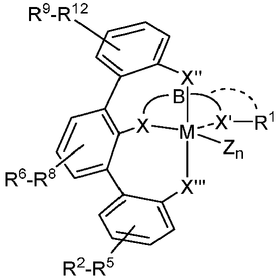 wherein
R²-R¹² include the same or different moieties selected from aromatic moieties, aliphatic moieties, heteroaromatic moieties, heteroaliphatic moieties, heteroatoms, substituted heteroatoms, or halide moieties; and
B has one to 15 atoms on shortest linkage path between X and X', not counting H or side groups.

5. The metal complex of any preceding claim, wherein the metal complex has the following structure: 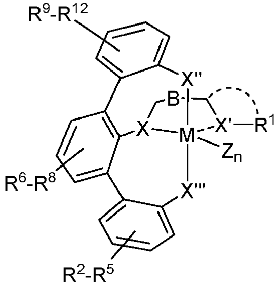 wherein
R²-R¹² include the same or different moieties selected from aromatic moieties, aliphatic moieties, heteroaromatic moieties, heteroaliphatic moieties, heteroatoms, substituted heteroatoms, or halide moieties; and
B has one to 13 atoms on shortest linkage path between X and X', not counting H or side groups.

6. The metal complex of any of claims 1 to 3, wherein the metal complex has the following structure: 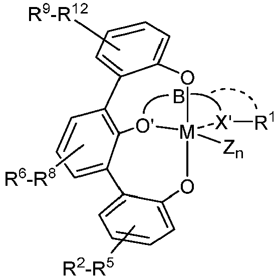 wherein
the two O-M bonds are covalent;
the O'-M bond is dative;
the X'-M bond is dative or does not exist;
R²-R¹² include the same or different moieties selected from aromatic moieties, aliphatic moieties, heteroaromatic moieties, heteroaliphatic moieties, heteroatoms, substituted heteroatoms, or halide moieties;
B has one to 10 atoms on shortest linkage path between O' and X', not counting H or side groups; and
Zₙ comprises 1-2 independent moieties that are either identical or different from one another, and wherein the Zₙ moieties form covalent bonds, dative bonds, ionic bonds, or combinations thereof with M.

7. The metal complex of any preceding claim, wherein X' is part of an aliphatic carbocycle, an aromatic carbocycle, a heterocyclic moiety, or a heteroaromatic moiety.

8. The metal complex of any preceding claim, wherein B is part of an aliphatic carbocycle, an aromatic carbocycle, or a heterocycle that connects onto R¹.

9. The metal complex of any preceding claim, wherein M comprises Zr or Hf.

10. The metal complex of any preceding claim, wherein the metal complex is a Cₛ-symmetrical meridional (mer) isomer.

11. A terphenyl ligand according to the following structure 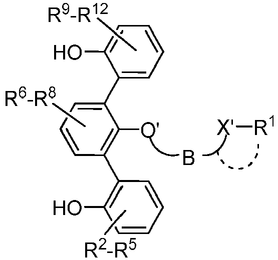 wherein
B is an aliphatic moiety or heteroaliphatic moiety linking O' and X', wherein B has from one to 50 atoms, not counting H; and
B has one to 10 atoms on shortest linkage path between O' and X', not counting H or side groups;
X' is oxygen, sulfur, substituted nitrogen, substituted phosphorus, or a substituted carbon;
R¹ is either: 1) an aliphatic moiety, a heteroaliphatic moiety, an aromatic moiety, or a heteroaromatic moiety; or 2) part of an aliphatic carbocycle, an aromatic carbocycle, a heterocyclic moiety, or a heteroaromatic moiety; and
R²-R¹² include the same or different moieties selected from aromatic moieties, aliphatic moieties, heteroaromatic moieties, heteroaliphatic moieties, heteroatoms or halide moieties;
wherein the terms "substituted nitrogen" is a tertiary, secondary or primary nitrogen moiety and "substituted phosphorus" is a tertiary, secondary or primary phosphorous moiety; and
the term "substituted carbon" is wherein at least one hydrogen atom bound to said carbon atom is replaced with one or more substituents that are functional groups selected from hydroxyl, alkoxyl, alkylthio, amino, halo and silyl.

12. The terphenyl ligand of claim 11, wherein X' is part of an aliphatic carbocycle, an aromatic carbocycle, a heterocyclic moiety, or a heteroaromatic moiety, and wherein B is part of an aliphatic carbocycle, an aromatic carbocycle, or a heterocycle that connects onto R¹.

## Patentansprüche

1. Ein Metallkomplex, der die folgende Struktur beinhaltet: 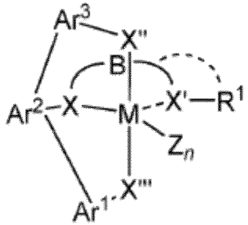 wobei
mindestens zwei der folgenden Bindungen X-M, X'-M, X"-M und X'''-M kovalent sind und mindestens eine dativ ist; die M-X'-Bindung kovalent ist, eine dative Bindung ist oder nicht existiert;
Ar¹, Ar² und Ar³ unabhängig ein Arylanteil oder ein Heteroarylanteil sind;
B ein aliphatischer Anteil oder ein heteroaliphatischer Anteil ist, der X und X' verknüpft, wobei B ein bis 50 Atome aufweist, wobei H nicht mitgezählt wird;
X Sauerstoff, Schwefel, substituierter Stickstoff, substituierter Phosphor oder substituierter Kohlenstoff ist;
X' Sauerstoff, Schwefel, substituierter Stickstoff, substituierter Phosphor oder eine ungesättigte Kohlenstoffgruppe ist;
X" und X''' unabhängig aus Sauerstoff, Schwefel, substituiertem Stickstoff oder substituiertem Phosphor ausgewählt sind,
R¹ entweder: 1) ein aliphatischer Anteil, ein heteroaliphatischer Anteil, ein aromatischer Anteil oder ein heteroaromatischer Anteil ist; oder 2) Teil eines aliphatischen Carbocyclus, eines aromatischen Carbocyclus, eines heterocyclischen Anteils oder eines heteroaromatischen Anteils ist; und
M ein oder mehrere Metalle ist, die aus einem Element der Gruppe 4 ausgewählt sind; wobei die Ausdrücke "substituierter Stickstoff" ein tertiärer, sekundärer oder primärer Stickstoffanteil ist und "substituierter Phosphor" ein tertiärer, sekundärer oder primärer Phosphoranteil ist; und
der Ausdruck "substituierter Kohlenstoff "Folgendes ist: wobei mindestens ein an das Kohlenstoffatom gebundenes Wasserstoffatom durch einen oder mehrere Substituenten ersetzt ist, die funktionelle Gruppen sind, die aus Hydroxyl, Alkoxyl, Alkylthio, Amino, Halogen und Silyl ausgewählt sind.

2. Metallkomplex gemäß Anspruch 1, wobei Ar¹ und Ar³ mit 1-4 Wasserstoffatomen, 1-4 aromatischen Anteilen, 1-4 aliphatischen Anteilen, 1-4 substituierten Heteroatomen oder 1-4 Halogenidanteilen substituiert sind und Ar² mit 1-3 Wasserstoffatomen, 1-3 aromatischen Anteilen, 1-3 aliphatischen Anteilen, 1-3 substituierten Heteroatomen oder 1-3 Halogenidanteilen substituiert ist.

3. Metallkomplex gemäß Anspruch 1 oder 2, wobei Zₙ 1-4 unabhängige Anteile umfasst, die entweder identisch oder voneinander verschieden sind, und wobei die Zₙ-Anteile kovalente Bindungen, dative Bindungen, lonenbindungen oder Kombinationen davon mit M bilden.

4. Metallkomplex gemäß einem der vorhergehenden Ansprüche, wobei der Metallkomplex die folgende Struktur aufweist: 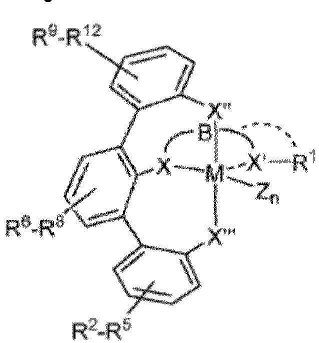 wobei
R²-R¹² die gleichen oder unterschiedliche Anteile umfassen, die aus aromatischen Anteilen, aliphatischen Anteilen, heteroaromatischen Aneilen, heteroaliphatischen Anteilen, Heteroatomen, substituierten Heteroatomen oder Halogenidanteilen ausgewählt sind; und
B ein bis 15 Atome auf dem kürzesten Verknüpfungsweg zwischen X und X', wobei H oder Seitengruppen nicht mitgezählt werden, aufweist.

5. Metallkomplex gemäß einem der vorhergehenden Ansprüche, wobei der Metallkomplex die folgende Struktur aufweist: 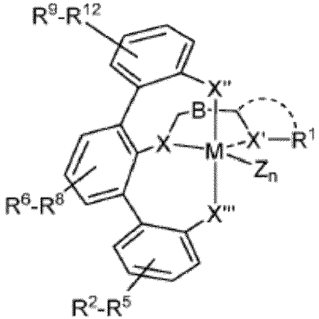 wobei
R²-R¹² die gleichen oder unterschiedliche Anteile umfassen, die aus aromatischen Anteilen, aliphatischen Anteilen, heteroaromatischen Aneilen, heteroaliphatischen Anteilen, Heteroatomen, substituierten Heteroatomen oder Halogenidanteilen ausgewählt sind; und
B ein bis 13 Atome auf dem kürzesten Verknüpfungsweg zwischen X und X', wobei H oder Seitengruppen nicht mitgezählt werden, aufweist.

6. Metallkomplex gemäß einem der Ansprüche 1 bis 3, wobei der Metallkomplex die folgende Struktur aufweist: 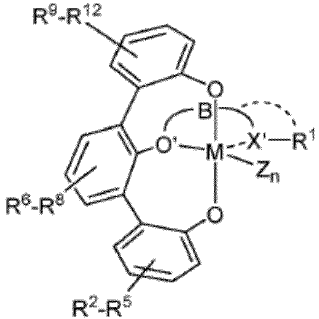 wobei
die zwei O-M-Bindungen kovalent sind;
die O'-M-Bindung dativ ist;
die X'-M-Bindung dativ ist oder nicht existiert;
R²-R¹² die gleichen oder unterschiedliche Anteile umfassen, die aus aromatischen Anteilen, aliphatischen Anteilen, heteroaromatischen Anteilen, heteroaliphatischen Anteilen, Heteroatomen, substituierten Heteroatomen oder Halogenidanteilen ausgewählt sind;
B ein bis 10 Atome auf dem kürzesten Verknüpfungsweg zwischen O' und X', wobei H oder Seitengruppen nicht mitgezählt werden, aufweist; und Zₙ 1-2 unabhängige Anteile umfasst, die entweder identisch oder voneinander verschieden sind, und wobei die Zₙ-Anteile kovalente Bindungen, dative Bindungen, lonenbindungen oder Kombinationen davon mit M bilden.

7. Metallkomplex gemäß einem der vorhergehenden Ansprüche, wobei X' Teil eines aliphatischen Carbocyclus, eines aromatischen Carbocyclus, eines heterocyclischen Anteils oder eines heteroaromatischen Anteils ist.

8. Metallkomplex gemäß einem der vorhergehenden Ansprüche, wobei B Teil eines aliphatischen Carbocyclus, eines aromatischen Carbocyclus oder eines Heterocyclus ist, der sich mit R¹ verbindet.

9. Metallkomplex gemäß einem der vorhergehenden Ansprüche, wobei M Zr oder Hf beinhaltet.

10. Metallkomplex gemäß einem der vorhergehenden Ansprüche, wobei der Metallkomplex ein Cₛ-symmetrisches meridionales (mer)lsomer ist.

11. Ein Terphenylligand gemäß der folgenden Struktur: 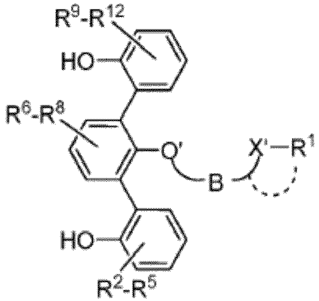 wobei
B ein aliphatischer Anteil oder ein heteroaliphatischer Anteil ist, der O' und X' verknüpft, wobei B ein bis 50 Atome aufweist, wobei H nicht mitgezählt wird; und
B ein bis 10 Atome auf dem kürzesten Verknüpfungsweg zwischen O' und X', wobei H oder Seitengruppen nicht mitgezählt werden, aufweist;
X' Sauerstoff, Schwefel, substituierter Stickstoff, substituierter Phosphor oder ein substituierter Kohlenstoff ist;
R¹ entweder: 1) ein aliphatischer Anteil, ein heteroaliphatischer Anteil, ein aromatischer Anteil oder ein heteroaromatischer Anteil ist; oder 2) Teil eines aliphatischen Carbocyclus, eines aromatischen Carbocyclus, eines heterocyclischen Anteils oder eines heteroaromatischen Anteils ist; und
R²-R¹² die gleichen oder unterschiedliche Anteile umfassen, die aus aromatischen Anteilen, aliphatischen Anteilen, heteroaromatischen Anteilen, heteroaliphatischen Anteilen, Heteroatomen oder Halogenidanteilen ausgewählt sind;
wobei die Ausdrücke "substituierter Stickstoff" ein tertiärer, sekundärer oder primärer Stickstoffanteil ist und "substituierter Phosphor" ein tertiärer, sekundärer oder primärer Phosphoranteil ist; und
der Ausdruck "substituierter Kohlenstoff" Folgendes ist: wobei mindestens ein an das Kohlenstoffatom gebundenes Wasserstoffatom durch einen oder mehrere Substituenten ersetzt ist, die funktionelle Gruppen sind, die aus Hydroxyl, Alkoxyl, Alkylthio, Amino, Halogen und Silyl ausgewählt sind.

12. Terphenylligand gemäß Anspruch 11, wobei X' Teil eines aliphatischen Carbocyclus, eines aromatischen Carbocyclus, eines heterocyclischen Anteils oder eines heteroaromatischen Anteils ist und wobei B Teil eines aliphatischen Carbocyclus, eines aromatischen Carbocyclus oder eines Heterocyclus ist, der sich mit R¹ verbindet.

## Revendications

1. Un complexe métallique comprenant la structure suivante : 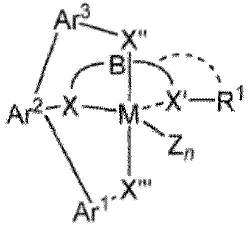 où
au moins deux des liaisons suivantes X-M, X'-M, X"-M, et X'''-M sont covalentes et au moins une est covalente de coordination ; la liaison M-X' est une liaison covalente de coordination, covalente ou n'existe pas ;
Ar¹, Ar², et Ar³ sont indépendamment un groupement aryle ou un groupement hétéroaryle ;
B est un groupement aliphatique ou un groupement hétéroaliphatique liant X et X', où B a de un à 50 atomes, sans compter H ;
X est de l'oxygène, du soufre, de l'azote substitué, du phosphore substitué, ou du carbone substitué ;
X' est de l'oxygène, du soufre, de l'azote substitué, du phosphore substitué, ou un groupe carbone insaturé ;
X" et X''' sont indépendamment sélectionnés parmi de l'oxygène, du soufre, de l'azote substitué, ou du phosphore substitué,
R¹ est soit : 1) un groupement aliphatique, un groupement hétéroaliphatique, un groupement aromatique, ou un groupement hétéroaromatique ; soit 2) une partie d'un carbocycle aliphatique, d'un carbocycle aromatique, d'un groupement hétérocyclique, ou d'un groupement hétéroaromatique ; et
M est un ou plusieurs métaux sélectionnés dans un élément de Groupe 4 ;
où le terme « azote substitué » est un groupement azote tertiaire, secondaire ou primaire et le terme « phosphore substitué » est un groupement phosphore tertiaire, secondaire ou primaire ; et
le terme « carbone substitué » est où au moins un atome d'hydrogène lié audit atome de carbone est remplacé par un ou plusieurs substituants qui sont des groupes fonctionnels sélectionnés parmi hydroxyle, alcoxyle, alkylthio, amino, halo et silyle.

2. Le complexe métallique de la revendication 1, où Ar¹ et Ar³ sont substitués par 1 à 4 hydrogènes, 1 à 4 groupements aromatiques, 1 à 4 groupements aliphatiques, 1 à 4 hétéroatomes substitués, ou 1 à 4 groupements halogénure, et Ar² est substitué par 1 à 3 hydrogènes, 1 à 3 groupements aromatiques, 1 à 3 groupements aliphatiques, 1 à 3 hétéroatomes substitués, ou 1 à 3 groupements halogénure.

3. Le complexe métallique des revendications 1 ou 2, où Zₙ inclut 1 à 4 groupements indépendants qui sont soit identiques, soit différents les uns des autres, et où les groupements Zₙ forment des liaisons covalentes, des liaisons covalentes de coordination, des liaisons ioniques, ou des combinaisons de celles-ci avec M.

4. Le complexe métallique de n'importe quelle revendication précédente, le complexe métallique ayant la structure suivante : 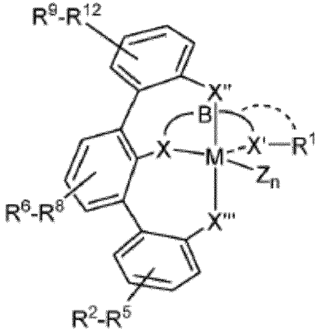 où
R² à R¹² incluent les mêmes groupements ou des groupements différents sélectionnés parmi des groupements aromatiques, des groupements aliphatiques, des groupements hétéroaromatiques, des groupements hétéroaliphatiques, des hétéroatomes, des hétéroatomes substitués, ou des groupements halogénure ; et
B a de un à 15 atomes sur une voie de liaison la plus courte entre X et X', sans compter H ou des groupes latéraux.

5. Le complexe métallique de n'importe quelle revendication précédente, le complexe métallique ayant la structure suivante : 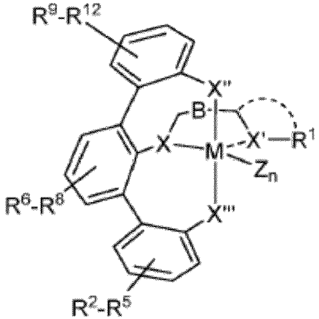 où
R² à R¹² incluent les mêmes groupements ou des groupements différents sélectionnés parmi des groupements aromatiques, des groupements aliphatiques, des groupements hétéroaromatiques, des groupements hétéroaliphatiques, des hétéroatomes, des hétéroatomes substitués, ou des groupements halogénure ; et
B a de un à 13 atomes sur une voie de liaison la plus courte entre X et X', sans compter H ou des groupes latéraux.

6. Le complexe métallique de n'importe lesquelles des revendications 1 à 3, le complexe métallique ayant la structure suivante : 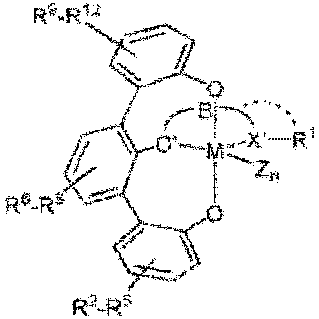 où
les deux liaisons O-M sont covalentes ;
la liaison O'-M est covalente de coordination ;
la liaison X'-M est covalente de coordination ou n'existe pas ;
R² à R¹² incluent les mêmes groupements ou des groupements différents sélectionnés parmi des groupements aromatiques, des groupements aliphatiques, des groupements hétéroaromatiques, des groupements hétéroaliphatiques, des hétéroatomes, des hétéroatomes substitués, ou des groupements halogénure ;
B a de un à 10 atomes sur une voie de liaison la plus courte entre O' et X', sans compter H ou des groupes latéraux ; et
Zₙ comprend 1 à 2 groupements indépendants qui sont soit identiques, soit différents l'un de l'autre, et où les groupements Zₙ forment des liaisons covalentes, des liaisons covalentes de coordination, des liaisons ioniques, ou des combinaisons de celles-ci avec M.

7. Le complexe métallique de n'importe quelle revendication précédente, où X' fait partie d'un carbocycle aliphatique, d'un carbocycle aromatique, d'un groupement hétérocyclique, ou d'un groupement hétéroaromatique.

8. Le complexe métallique de n'importe quelle revendication précédente, où B fait partie d'un carbocycle aliphatique, d'un carbocycle aromatique, ou d'un hétérocycle qui se raccorde sur R¹.

9. Le complexe métallique de n'importe quelle revendication précédente, où M comprend Zr ou Hf.

10. Le complexe métallique de n'importe quelle revendication précédente, le complexe métallique étant un isomère méridional (mer) de symétrie Cₛ.

11. Un ligand terphényle selon la structure suivante 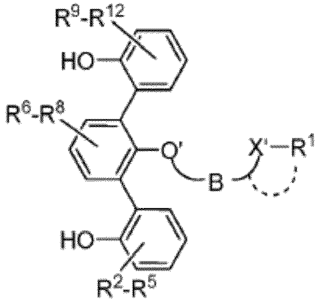 où
B est un groupement aliphatique ou un groupement hétéroaliphatique liant O' et X', où B a de un à 50 atomes, sans compter H ; et
B a de un à 10 atomes sur une voie de liaison la plus courte entre O' et X', sans compter H ou des groupes latéraux ;
X' est de l'oxygène, du soufre, de l'azote substitué, du phosphore substitué, ou un carbone substitué ;
R¹ est soit : 1) un groupement aliphatique, un groupement hétéroaliphatique, un groupement aromatique, ou un groupement hétéroaromatique ; soit 2) une partie d'un carbocycle aliphatique, d'un carbocycle aromatique, d'un groupement hétérocyclique, ou d'un groupement hétéroaromatique ; et
R² à R¹² incluent les mêmes groupements ou des groupements différents sélectionnés parmi des groupements aromatiques, des groupements aliphatiques, des groupements hétéroaromatiques, des groupements hétéroaliphatiques, des hétéroatomes ou des groupements halogénure ;
où le terme « azote substitué » est un groupement azote tertiaire, secondaire ou primaire et le terme « phosphore substitué » est un groupement phosphore tertiaire, secondaire ou primaire ; et
le terme « carbone substitué » est où au moins un atome d'hydrogène lié audit atome de carbone est remplacé par un ou plusieurs substituants qui sont des groupes fonctionnels sélectionnés parmi hydroxyle, alcoxyle, alkylthio, amino, halo et silyle.

12. Le ligand terphényle de la revendication 11, où X' fait partie d'un carbocycle aliphatique, d'un carbocycle aromatique, d'un groupement hétérocyclique, ou d'un groupement hétéroaromatique, et où B fait partie d'un carbocycle aliphatique, d'un carbocycle aromatique, ou d'un hétérocycle qui se raccorde sur R¹
